# EUROPEAN PATENT APPLICATION

(11) **EP 2 880 993 A1**
(43) Date of publication of application: **10.06.2015**
(21) Application number: 13382495.3
(22) Date of filing: 04.12.2013
(51) Int. Cl.: A23L 1/29, A61K 31/191, A61K 31/7016, A23L 1/308, A23L 1/30, A61P 25/28

(54) **Method of achieving memory and learning improvement by the administration of sialic acid**

(71) Applicant: ABBOTT LABORATORIES, Abbott Park, IL 60064-3500 (US)
(72) Inventor: MARTIN MARTIN, Maria Jesús, 18006 GRANADA (ES); RAMIREZ GONZALEZ, Maria, 18003 GRANADA (ES); OLIVEROS DELGADO, Elena, 18008 GRANADA (ES); BARRANCO PEREZ, Alejandro, 18110 LAS GABIAS (Granada) (ES); RUEDA CABRERA, Ricardo, 18110 LAS GABIAS (Granada) (ES)
(74) Representative: Boult Wade Tennant

(57) **Abstract**

Methods of achieving sustained memory improvements and sustained improvement in learning abilities in an infant are provided. The methods comprise administering an effective amount of sialic acid to an infant or a preterm infant until at least 90 days or three months after birth. The administration results in enhanced brain function, improved learning ability and sustained memory improvement in the infant.

## Description

### FIELD OF THE DISCLOSURE

The present disclosure relates to methods for improving the memory, learning abilities, or both, of an infant where the improvement is sustained at least until the infant reaches adolescence and in certain embodiments until the infant reaches adulthood. More particularly, the present disclosure relates to the use of an effective amount of sialic acid to enhance hippocampus function in the brain, resulting in sustained memory improvement, sustained improvement in learning abilities, or both, in the infant.

### BACKGROUND OF THE DISCLOSURE

Human breast milk is unique among mammals in the quantity of sialylated structures. Cow's milk is comparatively low in sialylated structures. The quantity of sialylated structures in human breast milk is high after parturition and progressively decreases near the end of lactation, suggesting that initially a growing brain requires higher amounts of sialic acid for cognitive development. The initially high levels of sialylated structures present in human breast milk also suggest that sialic acid found in sialylated structures demanded by a developing brain may be replenished by the sialylated components present in human breast milk.

Long term potentiation (LTP) is an electrophysiological measurement considered to be the basic mechanism of synaptic plasticity and memory formation. Previous scientific studies have linked LTP to the polysialylated embryonic form of the neural cell adhesion molecule (PSA-NCAM). *See e.g.,* Muller D, Wang C, Skibo G, et al,. PSA-NCAM Is Required for Activity-Induced Synoptic Plasticity, Neuron, 1996, 17:413-422; Eckhardt M, Bukalo O, Chazal G, et al., Mice Deficient in the Polysialyltransferase ST8SialV/PST-l Allow Discrimination of the Roles of Neural Cell Adhesion Molecule Protein And Polysialic acid in Neural Development and Synoptic Plasticity, J. Neurosci., 2000, 20:5234-5244. PSA-NCAM is a key protein of the brain and plays important roles in axonal outgrowth and neural plasticity. It is highly glycosialylated, bearing a structure rich in sialic acid moieties. PSA-NCAM may be the central sialylated protein involved in the prediction and enhancement of LTP.

### SUMMARY OF THE DISCLOSURE

The present disclosure is directed to methods of achieving sustained memory improvement or a sustained improvement in learning abilities by administration of sialic acid to premature infants and term infants.

A first embodiment is directed to a method of achieving sustained memory improvement in an infant. The method comprises administering an effective amount of sialic acid to the infant until at least 90 days after birth. According to the first embodiment, the amount of free sialic acid administered decreases as the infant ages. Further, in the first embodiment, the administration results in enhanced brain function in the infant and sustained memory improvement.

The first embodiment is also directed to sialic acid for use in enhancing brain function in an infant, wherein sialic acid is administered until at least 90 days after birth, and wherein the amount of sialic acid administered decreases as the infant ages. Preferably the enhanced brain function is sustained memory improvement.

The first embodiment is also directed to use of sialic acid in the manufacture of a medicament for use in enhancing brain function in an infant, wherein sialic acid is administered until at least 90 days after birth, and wherein the amount of sialic acid administered decreases as the infant ages. Preferably the enhanced brain function is sustained memory improvement.

In a second embodiment, a method of achieving sustained memory improvement is provided. The method comprises administering an effective amount of sialic acid to a preterm infant for at least 3 months after the birth of the infant, whereby the memory of the infant is improved and the improvement is sustained.

The second embodiment is also directed to sialic acid for use in improving memory in a preterm infant wherein memory improvement is sustained and wherein an effective amount of sialic acid is administered to the preterm infant for at least 3 months after the birth of the infant.

The second embodiment is also directed to use of sialic acid in the manufacture of a medicament for improving memory in a preterm infant wherein memory improvement is sustained and wherein an effective amount of sialic acid is administered to the preterm infant for at least 3 months after the birth of the infant.

In a third embodiment, a method of achieving sustained improvement in learning abilities in an infant is provided. The method comprises administering an effective amount of sialic acid to the infant for at least 3 months after the birth of the infant, whereby the learning abilities of the infant are improved and the improvement is sustained.

The third embodiment is also directed to sialic acid for use in improving learning abilities in an infant wherein the improvement is sustained and wherein an effective amount of sialic acid is administered to the infant for at least 3 months after the birth of the infant.

The third embodiment is also directed to use of sialic acid in the manufacture of a medicament for improving learning abilities in an infant wherein the improvement is sustained and wherein an effective amount of sialic acid is administered to the infant for at least 3 months after the birth of the infant.

In a fourth embodiment, a method of achieving sustained memory improvement in an infant is provided. The method comprises combining a composition having sialic acid with an infant formula that is low in sialoglycoconjugated compounds, and administering the combination to the infant, whereby the administration of the combination results in sustained memory improvement.

The fourth embodiment is also directed to a composition for use in improving memory in an infant, wherein the composition comprises sialic acid and an infant formula that is low in sialoglycoconjugated compounds, and wherein the improvement in memory is sustained. In one embodiment, the sialic acid is present as a composition comprising sialic acid.

The fourth embodiment is also directed to use of a composition in the manufacture of a medicament for improving memory in an infant, wherein the composition comprises sialic acid and an infant formula that is low in sialoglycoconjugated compounds, and wherein the improvement in memory is sustained. In one embodiment, the sialic acid is present as a composition comprising sialic acid.

### BRIEF DESCRIPTION OF THE DRAWINGS

FIG. 1 is a graph depicting the results of a memory retention test performed on rats according to Example 1.
FIG. 2 is a graph depicting the results of a memory test performed on rats, according to Example 1.
FIG. 3 is a graph depicting the results of LTP testing performed on rats, according to Example 1.
FIG. 4 is a graph depicting the results of LTP testing performed on rats, according to Example 2.
FIG. 5 is a graph depicting the results of Novel Object Recognition tests performed on rats, according to Example 2.
FIG. 6 is a graph depicting the results of a memory test performed on rats, according to Example 2.
FIG. 7 is a graph depicting the results of a memory test performed on rats, according to Example 2.
FIG. 8 is a graph depicting the results of an Intellicage® protocol performed on rats, according to Example 2.

### DETAILED DESCRIPTION OF THE DISCLOSURE

The methods described herein utilize sialic acid to achieve a sustained improvement in learning abilities and memory. These and other features of the methods (and the compositions, nutritional compositions, infant formulas useful therein), as well as some of the many optional variations and additions, are described in detail hereafter.

A first embodiment is directed to a method of achieving sustained memory improvement in an infant. The method comprises administering an effective amount of sialic acid to the infant until at least 90 days after birth. According to the first embodiment, the amount of free sialic acid administered decreases as the infant ages. Further, in the first embodiment, the administration results in enhanced brain function in the infant and sustained memory improvement.

The first embodiment is also directed to sialic acid for use in enhancing brain function in an infant, wherein sialic acid is administered until at least 90 days after birth, and wherein the amount of sialic acid administered decreases as the infant ages. Preferably the enhanced brain function is sustained memory improvement.

The first embodiment is also directed to the use of sialic acid in the manufacture of a medicament for enhancing brain function in an infant, wherein sialic acid is administered until at least 90 days after birth, and wherein the amount of sialic acid administered decreases as the infant ages. Preferably the enhanced brain function is sustained memory improvement.

In a second embodiment, a method of achieving sustained memory improvement is provided. The method comprises administering an effective amount of sialic acid to a preterm infant for at least 3 months after the birth of the infant, whereby the memory of the infant is improved and the improvement is sustained.

The second embodiment is also directed to sialic acid for use in improving memory in a preterm infant wherein memory improvement is sustained and wherein an effective amount of sialic acid is administered to the preterm infant for at least 3 months after the birth of the infant.

The second embodiment is also directed to use of sialic acid in the manufacture of a medicament for improving memory in a preterm infant wherein memory improvement is sustained and wherein an effective amount of sialic acid is administered to the preterm infant for at least 3 months after the birth of the infant.

In a third embodiment, a method of achieving sustained improvement in learning abilities in an infant is provided. The method comprises administering an effective amount of sialic acid to the infant for at least 3 months after the birth of the infant, whereby the learning abilities of the infant are improved and the improvement is sustained.

The third embodiment is also directed to sialic acid for use in improving learning abilities in an infant wherein the improvement is sustained and wherein an effective amount of sialic acid is administered to the infant for at least 3 months after the birth of the infant.

The third embodiment is also directed to use of sialic acid in the manufacture of a medicament for improving learning abilities in an infant wherein the improvement is sustained and wherein an effective amount of sialic acid is administered to the infant for at least 3 months after the birth of the infant.

In a fourth embodiment, a method of achieving sustained memory improvement in an infant is provided. The method comprises combining a composition having sialic acid with an infant formula that is low in sialoglycoconjugated compounds, and administering the combination to the infant, whereby the administration of the combination results in sustained memory improvement.

The fourth embodiment is also directed to a composition for use in improving memory in an infant, wherein the composition comprises sialic acid and an infant formula that is low in sialoglycoconjugated compounds, and wherein the improvement in memory is sustained. In one embodiment, the sialic acid is present as a composition comprising sialic acid.

The fourth embodiment is also directed to use of a composition in the manufacture of a medicament for improving memory in an infant, wherein the composition comprises sialic acid and an infant formula that is low in sialoglycoconjugated compounds, and wherein the improvement in memory is sustained. In one embodiment, the sialic acid is present as a composition comprising sialic acid.

The term "composition" as used herein should be understood to refer to compositions, including without limitation, infant formulas, supplements, and nutritional compositions.

The term "infant" as used herein, refers generally to persons less than 2 years of age, most typically less than 1 year of age regardless of whether the person was born prior to or after 36 weeks of gestation.

The term "preterm infant" as used herein, refers to a person born prior to 36 weeks of gestation.

The terms "full term infant" or "term infant" as used herein, refer to a person born after 36 weeks of gestation and preferably between 38 to 42 weeks of gestation.

The terms "child" and "childhood" as used herein, refer to a person greater than 2 years of age (i.e., not an infant) up to 10 years of age.

The terms "adult" and "adulthood" as used herein, refer to a person 18 years of age or older.

The terms "adolescence" as used herein, refer to a person greater than 10 years of age up to 18 years of age.

The terms "infant formula" or "synthetic infant formula" as used herein, unless otherwise specified, are used interchangeably and refer to liquid, solid, semi-liquid, and semi-solid human milk replacements or substitutes that are suitable for consumption by an infant. The synthetic formulas include components that are of semi-purified or purified origin. As used herein, unless otherwise specified, the terms "semi-purified" or "purified" refer to a material that has been prepared by purification of a natural material or by synthesis. The terms "infant formula" or "synthetic infant formula" do not include human breast milk or components from human breast milk unless otherwise indicated to the contrary.

The term "soy-based infant formula" as used herein, unless otherwise specified, refers to liquid, solid, semi-liquid, and semi-solid human milk replacements, infant formulas or substitutes that are made using soy protein and are suitable for consumption by an infant.

The term "mammalian food product" as used herein, unless otherwise specified, refers to formulas and other food products suitable for oral consumption by a human and having milk or dairy sourced from or secreted by mammals including, but not limited to: human, bovine, ovine, porcine, or caprine species.

The term "human milk fortifier" as used herein, unless otherwise specified, refers to liquid and solid nutritional products suitable for mixing with human breast milk or preterm infant formula or infant formula for consumption by a preterm or term infant.

The terms "susceptible" and "at risk" as used herein, unless otherwise specified, mean having little resistance to a certain condition or disease, including being genetically predisposed, having a family history of, and/or having symptoms of the condition or disease.

The terms "cognition" and "cognitive" as used herein, unless otherwise specified, refer to an individual's ability for learning, memory acquisition, and memory recall. In humans, testing and neuroimaging for hippocampus and brain function is well-known in the art and readily available to any psychologists, neuroradiologist or neuroscientist.

The term "free sialic acid" as used herein refers generally to the family of 9-carbon acidic sugar derivatives of neuraminic acid, specifically N- or O-substituted derivatives of neuraminic acid, most specifically but without limitation, N-Acetylneuraminic acid (Neu5Ac), and N-Glycolylneuraminic acid (Neu5Gc) including non-conjugated forms of sialic acid, as also detailed herein.

The term "conjugated sialic acid" as used herein refers generally to the family of 9-carbon acidic sugar derivatives of neuraminic acid, specifically conjugated forms of sialic acid, including without limitation protein-bound sialic acid, lipid-bound sialic acid (including gangliosides), carbohydrate-bound sialic acid, and combinations or derivatives thereof, including without limitation, 6'-sialyllactose, 3'-sialyllactose, 6'-sialyllactosamine and 3'-sialyllactosamine, among others.

The term "sialic acid" generally refers to all conjugated and non-conjugated forms of sialic acid, including sialic acid derivatives, free sialic acid, protein-bound sialic acid, lipid-bound sialic acid (including gangliosides), carbohydrate-bound sialic acid, and combinations or derivatives thereof.

The phrases "essentially free of sialoglycoconjugated compounds" or "essentially free of sialic acid" as used herein refers generally to less than 250 mg/L of sialic acid on an as fed basis.

The phrases "low in sialoglycoconjugated compounds" or "low in sialic acid" as used herein with respect to an infant formula refers generally to an amount of sialoglycoconjugated compounds or sialic acid that is less than the total amount of sialic acid present in sialylated structures found in normal human breast milk during a discrete time period after parturition, e.g., less than 1780 mg/L during the early stages of lactation or less than about 187 mg/L in mature human milk later in lactation. *See, e. g. ,* Am J Clin Nutr April 1985 vol. 41 no. 4 720-726 (disclosing mean concentrations of sialic acid during weeks 0-2 of lactation of 1138 +/- 86 mg/L, during weeks 2-4 of lactation 706 +/- 79 mg/L, during weeks 4-6 of lactation 348 +/- 63 mg/L, during weeks 6-8 of lactation 258 +/- 34 mg/L, and during weeks 10-28 of lactation 135 +/- 16 mg/L). Amounts below the mean amounts listed in the 1985 article should also be considered to be low for the respective times listed.

Numerical ranges as used herein are intended to include every number and subset of numbers within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The compositions useful in the methods of the present disclosure may be formulated and administered in any known or otherwise suitable oral or enteral product form. Generally, any solid, liquid, semi-solid, semi-liquid or powder product form, including combinations or variations thereof, are suitable for use in the methods disclosed herein, provided that such forms allow for safe and effective oral, or enteral delivery to the individual of the essential ingredients and any optional ingredients, as also defined herein. Powders and concentrated liquids utilized in the present disclosure are intended to be reconstituted into a liquid for safe consumption by an infant.

### Product Forms

The compositions suitable for use in the methods according to the first, second, third and fourth embodiments of the present disclosure (i.e., compositions by which the sialic acid is administered to the infant) may be formulated and administered in any known or otherwise suitable oral product form. Any solid, semi-solid, liquid, semi-liquid, or powder product form, including combinations or variations thereof, are suitable for use herein, provided that such forms allow for safe and effective oral delivery to the individual of the essential ingredients and any optional ingredients, according to the methods of the first, second, third and fourth embodiments as discussed herein.

The compositions suitable for use in the methods according to the first, second, third and fourth embodiments of the present disclosure (i.e., compositions by which the sialic acid is administered to the infant) are desirably formulated as nutritional composition product forms, which are defined herein as comprising the ingredients of the present disclosure in a product form that contains at least one of fat, protein, and carbohydrate, and preferably also vitamins, minerals, or combinations thereof. In certain embodiments according to the methods of the first, second, third and fourth embodiments disclosed herein, the nutritional compositions utilized (e.g., infant formulas) comprise sialic acid, desirably in combination with at least one of protein, fat, carbohydrates, vitamins, and minerals, to produce a nutritional composition.

In certain embodiments of the methods of the first, second, third and fourth embodiments disclosed herein, the nutritional composition (e.g., infant formula) may be formulated with sufficient kinds and amounts of nutrients to provide a sole, primary, or supplemental source of nutrition, or to provide a specialized nutritional composition for use in individuals (e.g., infants) afflicted with specific diseases, disorders, or conditions.

Specific non-limiting examples of product forms suitable for use in certain embodiments of the methods of the first, second, third and fourth embodiments disclosed herein, include liquid and powdered human milk fortifiers, liquid and powdered preterm infant formulas, and liquid and powdered infant formulas. By way of non-limiting examples, these product forms may be administered orally via a bottle, a syringe, dropper, droplets, or any other administering device or method known in the art.

### Infant Formulas and Human Milk Fortifiers

As previously mentioned, in certain embodiments of the methods of the first, second, third and fourth embodiments disclosed herein, the sialic acid may be administered via an infant formula or human milk fortifier. Exemplary compositions suitable for administering the sialic acid according to certain embodiments of the methods of the first, second, third and fourth embodiments disclosed herein, include both concentrated and ready-to-feed nutritional liquids. These nutritional liquids are most typically formulated as suspensions or emulsions, although other liquid forms are within the scope of the present disclosure.

Nutritional emulsions suitable for use in administering the sialic acid according to certain embodiments of the methods of the first, second, third and fourth embodiments disclosed herein, may be aqueous emulsions comprising protein, fat, and carbohydrate, vitamins, minerals and other nutrients helpful for optimal growth and development. These emulsions are generally flowable or drinkable liquids at temperatures from 1°C to 25°C and are typically in the form of oil-in-water, water-in-oil, or complex aqueous emulsions, although such emulsions are most typically in the form of oil-in-water emulsions having a continuous aqueous phase and a discontinuous oil phase.

Nutritional emulsions suitable for use in administering the sialic acid according to certain embodiments of the methods of the first, second, third and fourth embodiments disclosed herein, may be and typically are shelf stable. Such nutritional emulsions typically contain up to 95% by weight of water, including from 50% to 95%, also including from 60% to 90%, and also including from 70% to 85%, by weight of water. Such nutritional emulsions may have a variety of product densities, but most typically have a density greater than 1.03 g/mL, including greater than 1.04 g/mL, including greater than 1.055 g/mL, including from 1.03 g/mL to 1.12 g/mL, including from 1.06 g/mL to 1.12 g/mL, and also including from 1.085 g/mL to 1.10 g/mL.

Nutritional emulsions suitable for use in administering the sialic acid according to certain embodiments of the methods of the first, second, third and fourth embodiments disclosed herein, may have a caloric density tailored to the nutritional needs of the ultimate consumer (i.e., the infant), although in most instances the nutritional emulsions comprise generally at least 19 kcal/fl oz (660 kcal/liter), more typically from 20 kcal/fl oz (675-680 kcal/liter) to 25 kcal/fl oz (820 kcal/liter), even more typically from 20 kcal/fl oz (675-680 kcal/liter) to 24 kcal/fl oz (800-810 kcal/liter). Generally, the 22-24 kcal/fl oz formulas are more commonly used in preterm or low birth weight infants, and the 20-21 kcal/fl oz (675-680 to 700 kcal/liter) formulas are more often used in full term infants. In some embodiments, the nutritional emulsion may have a caloric density of from 50 - 100 kcal/liter to 660 kcal/liter, including from 150 kcal/liter to 500 kcal/liter. In some specific embodiments of the methods of the first, second, third and fourth embodiments disclosed herein, the nutritional emulsion may have a caloric density of about 25, or 50, or 75, or 100 kcal/liter.

Nutritional emulsions suitable for use in administering the sialic acid according to certain embodiments of the methods of the first, second, third and fourth embodiments disclosed herein, may have a pH ranging from 3.5 to 8, but are most advantageously in a range from 4.5 to 7.5, including from 5.5 to 7.3, including from 6.2 to 7.2.

Although the serving size for a nutritional emulsion can vary depending upon a number of variables, a typical serving size suitable for use in certain embodiments of the methods of the first, second, third and fourth embodiments disclosed herein, is generally at least 1 mL, or even at least 2 mL, or even at least 5 mL, or even at least 10 mL, or even at least 25 mL, including ranges from 2 mL to 300 mL, including from 4 mL to 250 mL, and including from 10 mL to 240 mL.

### Nutritional Solids

As previously mentioned, in certain embodiments according to the methods of the first, second, third and fourth embodiments disclosed herein, the sialic acid is administered via a nutritional solid. Such nutritional solids may be in any solid form, but are typically in the form of flowable or substantially flowable particulate compositions, or at least particulate compositions. Particularly suitable nutritional solid product forms for use in certain embodiments according to the methods of the first, second, third, and fourth embodiments disclosed herein include spray dried, agglomerated and/or dryblended powder compositions. Such compositions can easily be scooped and measured with a spoon or similar other device, and can easily be reconstituted by the intended user with a suitable aqueous liquid, typically water, to form a nutritional composition for immediate oral or enteral use. In this context, "immediate" use generally means within about 48 hours, most typically within about 24 hours, preferably within about 1-2 hours after reconstitution.

Nutritional powders suitable for use in certain embodiments of the methods of the first, second, third and fourth embodiments disclosed herein, may be reconstituted with water prior to use (i.e., prior to administration to the infant) to a caloric density tailored to the nutritional needs of the ultimate user, although in most instances the powders are reconstituted with water to form compositions comprising at least 19 kcal/fl oz (660 kcal/liter), more typically from 20 kcal/fl oz (675-680 kcal/liter) to 25 kcal/fl oz (820 kcal/liter), even more typically from 20 kcal/fl oz (675-680 kcal/liter) to 24 kcal/fl oz (800-810 kcal/liter). Generally, the 22-24 kcal/fl oz formulas are more commonly used in preterm or low birth weight infants, and the 20-21 kcal/fl oz (675-680 to 700 kcal/liter) formulas are more often used in full term infants. In some embodiments, the reconstituted powder may have a caloric density of from 50-100 kcal/liter to 660 kcal/liter, including from 150 kcal/liter to 500 kcal/liter. In some specific embodiments, the emulsion may have a caloric density of about 25, or 50, or 75, or 100 kcal/liter.

### Sialic Acid

Compositions suitable for use in certain embodiments of the methods according to the first, second, third and fourth embodiments disclosed herein comprise sialic acid at a concentration on an as-fed basis of at least 500 mg/L, including from 500 mg/L to 1500 mg/L, also including from 500 mg/L to 2000 mg/L. It should be understood that the specific amounts of sialic acid discussed in this paragraph and the following paragraphs of this section (i.e., both the concentrations in terms of mg/L and the amounts administered in terms of mg/kg body weight) are intended to provide examples of the effective amounts of sialic acid that may be administered according to the methods of the first, second, third, and fourth embodiments disclosed herein.

Compositions suitable for use in certain embodiments of the methods according to the first, second, third and fourth embodiments disclosed herein include sialic acid in the form of free sialic acid; derivatives of neuraminic acid, specifically N- or O-substituted derivatives of neuraminic acid, most specifically but without limitation, N-Acetylneuraminic acid (Neu5Ac), and N-Glycolylneuraminic acid (Neu5Gc); conjugated sialic acid, specifically including without limitation protein-bound sialic acid, lipid-bound sialic acid (including gangliosides), carbohydrate-bound sialic acid, protein conjugates, lipid conjugates, oligosaccharide conjugates, and combinations or derivatives thereof, including without limitation, 6'-sialyllactose, 3'-sialyllactose, 6'-sialyllactosamine and 3'-sialyllactosamine, and casein glycomacropeptide. In certain embodiments according to the methods of the first, second, third, and fourth embodiments disclosed herein, the sialic acid that is administered is selected from the group consisting of free sialic acid, 6'-sialyllactose, 3'-sialyllactose, 6'-sialyllactosamine and 3'-sialyllactosamine, among others, and combinations thereof. In certain embodiments according to the methods of the first, second, third, and fourth embodiments disclosed herein, the sialic acid that is administered is selected from the group consisting of free sialic acid, 6'-sialyllactose, and combinations thereof. In certain embodiments according to the methods of the first, second, third, and fourth embodiments disclosed herein, the sialic acid that is administered is 6'-sialyllactose. In certain embodiments according to the methods of the first, second, third, and fourth embodiments disclosed herein, the sialic acid that is administered is free sialic acid. In certain embodiments of the methods according to the first, second, third, and fourth embodiments disclosed herein, the sialic acid (e.g., free sialic acid, 6'-sialyllactose, 3'-sialyllactose, 6'-sialyllactosamine and 3'-sialyllactosamine, and combinations thereof) is synthetic. Various embodiments according to the methods of the first, second, third and fourth embodiments disclosed herein can utilize any of the types or forms of sialic acid identified above, including without limitation combinations of several or each form herein disclosed.

According to the first embodiment of the methods disclosed herein, the amount of sialic acid administered decreases as the infant ages. In other embodiments according to the first embodiment of the methods disclosed herein, the amount of sialic acid administered decreases as the infant ages on a week-to-week basis, a month-to-month basis, a bi-monthly to bi-monthly basis, or according to some other time period. In certain embodiments, this decrease in the amount of sialic acid administered may be achieved by administration of compositions (e.g., infant formulas) having decreasing concentrations of sialic acid. In certain embodiments according to the first embodiment of the methods disclosed herein, the concentration of sialic acid in the composition administered to the infant (e.g., infant formula), on an as-fed basis, may be at least 1500 mg/L through day 5 postpartum, including from 1000 mg/L to 2000 mg/L. In certain embodiments according to the first embodiment of the methods disclosed herein, the concentration of sialic acid in the composition administered to the infant (e.g., an infant formula), on an as-fed basis, may be at least 1000 mg/L after day 5 postpartum through day 15 postpartum, including from 500 mg/L to 1500 mg/L. In certain embodiments according to the first embodiment of the methods disclosed herein, the concentration of sialic acid in the composition administered to the infant (e.g., an infant formula), on an as-fed basis, may be at least 500 mg/L after day 15 postpartum through the third or the sixth month postpartum, including from 200 mg/L to 1000 mg/L. Likewise, in certain embodiments of the second, third, and fourth embodiments disclosed herein, the amount of sialic acid in the compositions, nutritional composition, or infant formulas administered to the infant decreases as the infant ages in the manner described in the preceding sentences.

In certain embodiments of the methods of the first, second, third and fourth embodiments disclosed herein, the sialic acid is administered on an as-fed basis of at least 60 mg/kg bodyweight (of the infant)/day, including from 60 mg/kg bodyweight (of the infant)/day, to 250 mg/kg bodyweight (of the infant)/day, also including from 60 mg/kg bodyweight (of the infant)/day to 400 mg/kg bodyweight (of the infant)/day. In certain embodiments, the sialic acid is administered via an infant formula having a concentration of free sialic acid as discussed in the preceding sentence and paragraphs.

According to the first embodiment, the amount of sialic acid administered decreases on as the infant ages, optionally on a week-to-week, a month-to-month, or a bi-monthly to bi-monthly basis. In certain such embodiments, the decrease is measured in terms of the total amount of sialic acid administered per day according to the infant's body weight. In certain such embodiments, the amount of sialic acid, on an as-fed basis, may be at least 250 mg/kg bodyweight (of the infant)/day, through day 5 postpartum, including from 200 mg/kg bodyweight (of the infant)/day to 400 mg/kg bodyweight (of the infant)/day. In certain embodiments of the first embodiment, the amount of sialic acid administered, on an as-fed basis, may be at least 200 mg/kg bodyweight (of the infant)/day after day 5 postpartum through day 15 postpartum, including from 60 mg/kg bodyweight (of the infant) /day to 250 mg/kg bodyweight (of the infant)/day. In certain embodiments of the first embodiment, the amount of sialic acid, on an as-fed basis, may be at least 60 mg/kg bodyweight of the infant)/day after day 15 postpartum through 3 months or 6 months postpartum, including from 60 mg/kg bodyweight (of the infant)/day to 100 mg/kg bodyweight (of the infant)/day. Likewise, in certain embodiments of the second, third, and fourth embodiments disclosed herein, the amount of sialic acid administered (e.g., via a composition, nutritional composition, or infant formula) decreases as the infant ages in the manner described in the preceding sentences.

Additionally, in certain embodiments of the first, second, third, and fourth embodiments disclosed herein, the total amount of sialic acid administered to the infant may be roughly equal to the total amount of sialic acid present in sialylated structures found in normal human breast milk during the first 6 months of lactation, which can vary from 1780 mg/L during the early stages of lactation to 187 mg/L in mature milk, including from 500 mg/L to 1500 mg/L. For the avoidance of any doubt, as used herein the term "normal" human breast milk means breast milk that includes sialic acid in an amount of 187 mg/L to 1780 mg/L. *See e.g.,* Am J Clin Nutr October 2001 vol. 74 no. 4 510-515; Am J Clin Nutr April 1985 vol. 41 no. 4 720-726. For example, the total amount of sialic acid administered to the infant in the first 5 days, the period after day 5 through day 15, the period after day 15 and thereon, may be customized to mimic the average amount of sialic acid present in human breast milk during the relative time period. In still other embodiments of the first, second, third, and fourth embodiments disclosed herein, the total amount of sialic acid administered to the infant is intended to be somewhat less than the total amount present in normal human breast milk during the first 6 months of lactation.

In certain embodiments of the first, second, third, and fourth embodiments disclosed herein, sialic acid may be obtained from and added as a separate ingredient to existing commercial infant formula, in which case the added sialic acid is combined with any inherent sialic acid from other ingredients to provide the total sialic acid content in the infant formula.

In certain embodiments according to the methods of the first, second, third, and fourth embodiments, the sialic acid that is administered is an acidic oligosaccharide. Acidic oligosaccharides are human milk oligosaccharides with the monomer sialic acid linked to them. The acidic oligosaccharides are known as sialyloligosaccharides. Thirty different structures containing NeuAc have been identified to date in human milk, from simple trisaccharides as 3'SL to very complex structures, Wy et al, J Proteome 10;2011:856-868. In certain embodiments according to the methods of the first, second, third, and fourth embodiments, the sialic acid is selected from the group consisting of 3'-sialyllactose, 6'-sialyllactose, and combinations thereof.

In certain embodiments according to the methods of the first, second, third, and fourth embodiments disclosed herein, the sialic acid is administered to the infant in combination with other components, e.g., prebiotic oligosaccharides, probiotics, etc. In many embodiments according to the methods of the first, second, third, and fourth embodiments disclosed herein, the sialic acid is administered to the infant in a nutritional composition with multiple additional components, e.g. an infant formula. Generally, sialic acid suitable for use in the methods of the first, second, third, and fourth embodiments disclosed herein may be produced via fermentation, chemical synthesis, or combinations thereof.

All sialic acid concentrations described herein are based upon the weight percentage of the sialic acid compound or moiety itself, less protein, lipid, carbohydrate, or other conjugates bound to the sialic acid structure.

### Free Sialic Acid

In certain embodiments of the methods of the first, second, third, and fourth embodiments disclosed herein, the sialic acid that is administered is free sialic acid. In certain such embodiments, compositions suitable for use in the methods may comprise free sialic acid at a concentration on an as-fed basis of at least 500 mg/L, including from 500 mg/L to 1500 mg/L, also including from 500 mg/L to 2000 mg/L. It should be understood that the specific amounts of free sialic acid discussed in this paragraph and the following paragraphs of this section (i.e., both the concentrations in terms of mg/L and the amounts administered in terms of mg/kg body weight) are intended to provide examples of the effective amount of free sialic acid that may be administered according to the methods of the first, second, third, and fourth embodiments disclosed herein.

In certain embodiments according to methods of the first embodiment, the sialic acid is free sialic acid and the amount of free sialic acid administered decreases as the infant ages, optionally on a week-to-week, a month-to-month, or a bi-monthly to bi-monthly basis. In certain such embodiments, this decrease in the amount of free sialic acid administered may be achieved by administration of compositions (e.g., infant formulas) having decreasing concentrations of free sialic acid. In such embodiments, the concentration of free sialic acid, on an as-fed basis, may be at least 1500 mg/L through day 5 postpartum, including from 1000 mg/L to 2000 mg/L. In such embodiments, the concentration of free sialic acid, on an as-fed basis, may be at least 1000 mg/L after day 5 postpartum through day 15 postpartum, including from 500 mg/L to 1500 mg/L. In such embodiments, the concentration of free sialic acid, on an as-fed basis, may be at least 500 mg/L after day 15 postpartum through 3 months or 6 months postpartum, including from 200 mg/L to 1000 mg/L. Likewise, in certain embodiments of the second, third, and fourth embodiments disclosed herein, the amount of free sialic acid in the compositions, supplements, and infant formulas administered decreases as the infant ages in the manner as described in the preceding sentences.

In certain embodiments of the first, second, third, and fourth embodiments disclosed herein, the compositions suitable for use in the methods of the present disclosure comprise free sialic acid at a concentration on an as-fed basis of at least 60 mg/kg bodyweight (of the infant)/day, including from 60 mg/kg bodyweight (of the infant)/day, to 250 mg/kg bodyweight (of the infant)/day, also including from 60 mg/kg bodyweight (of the infant)/day to 400 mg/kg bodyweight (of the infant)/day.

In certain embodiments according to the first embodiment, the sialic acid is free sialic acid, and the amount of free sialic acid administered decreases as the infant ages, optionally on a week-to-week, a month-to-month, or a bi-monthly to bi-monthly basis. In certain such embodiments, the decrease is measured in terms of the total amount of free sialic acid administered per day according to the infant's body weight. In certain such embodiments, the amount of free sialic acid, on an as-fed basis, may be at least 250 mg/kg bodyweight (of the infant)/day through day 5 postpartum, including from 200 mg/kg bodyweight (of the infant)/day to 400 mg/kg bodyweight (of the infant)/day. In certain such embodiments, the amount of free sialic acid administered, on an as-fed basis, may be at least 200 mg/kg bodyweight (of the infant)/day after day 5 postpartum through day 15 postpartum, including from 60 mg/kg bodyweight (of the infant) /day to 250 mg/kg bodyweight (of the infant)/day. In certain embodiments of the first embodiment, the amount of free sialic acid, on an as-fed basis, may be at least 60 mg/kg bodyweight (of the infant)/day after day 15 postpartum through 3 months or 6 months postpartum, including from 60 mg/kg bodyweight (of the infant)/day to 100 mg/kg bodyweight (of the infant)/day. Likewise, in certain embodiments of the second, third, and fourth embodiments disclosed herein, the amount of free sialic acid in the compositions, nutritional composition, and infant formulas administered decreases as the infant ages in the manner described in the immediately preceding sentences.

Additionally, in certain embodiments of the first, second, third, and fourth embodiments disclosed herein, the total amount of free sialic acid administered to the infant may be roughly equal to the total amount of sialic acid present in sialylated structures found in normal human breast milk during the first 6 months of lactation, including from 500 mg/L to 1500 mg/L. For the avoidance of any doubt, the amount of sialic acid in "normal" human breast milk, as that term is used herein, is 500 mg/L to 1500 mg/L. In still other embodiments of the first, second, third, and fourth embodiments disclosed herein, the total amount of free sialic acid administered to the infant is intended to be somewhat less than the total amount present in normal human breast milk during the first 6 months of lactation.

In certain embodiments of the first, second, third, and fourth embodiments disclosed herein, the free sialic acid may be obtained from and added as a separate ingredient to the infant formula that is administered to an infant, in which case the added free sialic acid is combined with any inherent sialic acid from other ingredients to provide the total sialic acid content in the infant formula.

As an individual compound or moiety, free sialic acid is a 9 carbon amino sugar, the structure of which is readily described in the chemical literature. Other generally accepted names for free sialic acid include N-acetylneuraminic acid; o-Sialic acid; 5-Acetamido-3,5-dideoxy-D-glycero-D-galacto-2-nonulosonic acid; 5-Acetamido-3,5-dideoxy-D-glycero-D-galactonulosonic acid; N-acetyl-neuraminate; N-Acetylneuraminic acid; NANA; and Neu5Ac.

Additionally, in certain embodiments of the first, second, third, and fourth embodiments disclosed herein, the term free sialic acid excludes oligosaccharide conjugates, lipid conjugates, protein conjugates and combinations thereof and the compositions are free of oligosaccharide conjugates, lipid conjugates, protein conjugates and combinations thereof; in such embodiments, the term free sialic acid may exclude lipid bound sialic acid, including gangliosides, carbohydrate-bound sialic acid, and protein-bound sialic acid. Additionally, in certain embodiments of the first, second, third, and fourth embodiments disclosed herein, the term free sialic acid may exclude 3'sialyllactose, and casein glycomacropeptide.

In certain embodiments of the methods according to the first, second, third, and fourth embodiments disclosed herein, the free sialic acid is administered in nutritional compositions in combination with other components, e.g., prebiotic oligosaccharides, probiotics, etc. In certain embodiments of the methods of the first, second, third, and fourth embodiments disclosed herein, the free sialic acid is included in nutritional compositions with multiple additional components. Suitable free sialic acid for use in the methods of the first, second, third, and fourth embodiments disclosed herein may be produced via fermentation, chemical synthesis, or combinations thereof.

### 6'-sialyllactose

In certain embodiments of the methods of the first, second, third, and fourth embodiments disclosed herein, the sialic acid that is administered is 6'-sialyllactose. In certain such embodiments, compositions suitable for use in the methods may comprise 6'-sialyllactose at a concentration on an as-fed basis of at least 500 mg/L, including from 500 mg/L to 1500 mg/L, also including from 500 mg/L to 2000 mg/L. It should be understood that the specific amounts of 6'-sialyllactose discussed in this paragraph and the following paragraphs of this section (i.e., both the concentrations in terms of mg/L and the amounts administered in terms of mg/kg body weight) are intended to provide examples of the effective amount of 6'-sialyllactose that may be administered according to the methods of the first, second, third, and fourth embodiments disclosed herein.

In certain embodiments according to methods of the first embodiment, the sialic acid is 6'-sialyllactose and the amount of 6'-sialyllactose administered decreases as the infant ages, optionally on a week-to-week, a month-to-month, or a bi-monthly to bi-monthly basis. In certain such embodiments, this decrease in the amount of 6'-sialyllactose administered may be achieved by administration of compositions (e.g., infant formulas) having decreasing concentrations of 6'-sialyllactose. In such embodiments, the concentration of 6'-sialyllactose, on an as-fed basis, may be at least 1500 mg/L through day 5 postpartum, including from 1000 mg/L to 2000 mg/L. In such embodiments, the concentration of 6'-sialyllactose, on an as-fed basis, may be at least 1000 mg/L after day 5 postpartum through day 15 postpartum, including from 500 mg/L to 1500 mg/L. In such embodiments, the concentration of 6'-sialyllactose, on an as-fed basis, may be at least 500 mg/L after day 15 postpartum through 3 months or 6 months postpartum, including from 200 mg/L to 1000 mg/L. Likewise, in certain embodiments of the second, third, and fourth embodiments disclosed herein, the amount of 6'-sialyllactose in the compositions, supplements, and infant formulas administered decreases as the infant ages in the manner as described in the preceding sentences.

In certain embodiments of the first, second, third, and fourth embodiments disclosed herein, the compositions suitable for use in the methods of the present disclosure comprise 6'-sialyllactose at a concentration on an as-fed basis of at least 60 mg/kg bodyweight (of the infant)/day, including from 60 mg/kg bodyweight (of the infant)/day to 250 mg/kg bodyweight (of the infant)/day, also including from 60 mg/kg bodyweight (of the infant)/day to 400 mg/kg bodyweight (of the infant) /day.

In certain embodiments according to the first embodiment, the sialic acid is 6'-sialyllactose, and the amount of 6'-sialyllactose administered decreases as the infant ages, optionally on a week-to-week, a month-to-month, or a bi-monthly to bi-monthly basis as the infant ages. In certain such embodiments, the decrease is measured in terms of the total amount of free sialic acid administered per day according to the infant's body weight. In certain such embodiments, the amount of 6'-sialyllactose, on an as-fed basis, may be at least 250 mg/kg bodyweight (of the infant)/day through day 5 postpartum, including from 200 mg/kg bodyweight (of the infant)/day to 400 mg/kg bodyweight (of the infant)/day. In certain such embodiments, the amount of 6'-sialyllactose administered, on an as-fed basis, may be at least 200 mg/kg bodyweight (of the infant)/day after day 5 postpartum through day 15 postpartum, including from 60 mg/kg bodyweight (of the infant)/day to 250 mg/kg bodyweight (of the infant)/day. In certain embodiments of the first embodiment, the amount of 6'-sialyllactose, on an as-fed basis, may be at least 60 mg/kg bodyweight (of the infant)/day after day 15 postpartum through 3 months or through 6 months postpartum, including from 60 mg/kg bodyweight (of the infant) /day to 100 mg/kg bodyweight (of the infant)/day. Likewise, in certain embodiments of the second, third, and fourth embodiments disclosed herein, the amount of 6'-sialyllactose in the compositions, nutritional composition, and infant formulas administered decreases as the infant ages in the manner described in the immediately preceding sentences.

Additionally, in certain embodiments of the first, second, third, and fourth embodiments disclosed herein, the total amount of 6'-sialyllactose administered to the infant may be roughly equal to the total amount of sialic acid present in sialylated structures found in normal human breast milk during the first 6 months of lactation, including from 500 mg/L to 1500 mg/L. For the avoidance of any doubt, the amount of sialic acid in "normal" human breast milk, as that term is used herein, is 500 mg/L to 1500 mg/L. In still other embodiments of the first, second, third, and fourth embodiments disclosed herein, the total amount of 6'-sialyllactose administered to the infant is intended to be somewhat less than the total amount present in normal human breast milk during the first 6 months of lactation.

In certain embodiments of the first, second, third, and fourth embodiments disclosed herein, the 6'-sialyllactose may be obtained from and added as a separate ingredient to the infant formula that is administered to an infant, in which case the added 6'-sialyllactose is combined with any inherent sialic acid from other ingredients to provide the total sialic acid content in the infant formula.

As an individual compound or moiety, 6'-sialyllactose has the molecular formula C₂₃H₃₉NO₁₉, a molecular weight of 633.55106 Daltons and is represented by the chemical structure in Equation (1). In 6'-sialyllactose, the N-acetylneuraminyl (NeuAc) unit is connected to the galactosyl unit of lactose at the 6 position. 6'-sialyllactose is often abbreviated as 6'-SL and is also known by other names such as 6'-α-Sialyllactose, 6'-Monosialyllactose, (2>6')-Sialyllactose, (2>6')-a-Sialyllactose, 6'-alpha-Sialyllactose, and (2>6')-alpha-Sialyllactose. 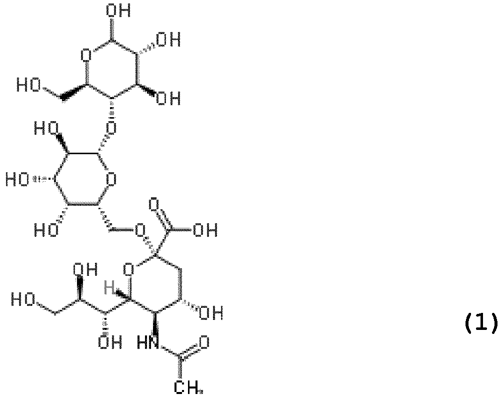 In certain embodiments of the methods according to the first, second, third, and fourth embodiments disclosed herein, the 6'-sialyllactose is administered in nutritional compositions in combination with other components, e.g., prebiotic oligosaccharides, probiotics, etc. In certain embodiments of the methods of the first, second, third, and fourth embodiments disclosed herein, the 6'-sialyllactose is included in a nutritional composition with multiple additional components. Suitable 6'-sialyllactose for use in the methods of the first, second, third, and fourth embodiments disclosed herein may be produced via fermentation, chemical synthesis, or combinations thereof.

### 3'-sialyllactose

In certain embodiments of the methods of the first, second, third, and fourth embodiments disclosed herein, the sialic acid that is administered is 3'-sialyllactose. In certain such embodiments, compositions suitable for use in the methods may comprise 3'-sialyllactose at a concentration on an as-fed basis of at least 500 mg/L, including from 500 mg/L to 1500 mg/L, also including from 500 mg/L to 2000 mg/L. It should be understood that the specific amounts of 3'-sialyllactose discussed in this paragraph and the following paragraphs of this section (i.e., both the concentrations in terms of mg/L and the amounts administered in terms of mg/kg body weight) are intended to provide examples of the effective amount of 3'- sialyllactose that may be administered according to the methods of the first, second, third, and fourth embodiments disclosed herein.

In certain embodiments according to methods of the first embodiment, the sialic acid is 3'-sialyllactose and the amount of 3'-sialyllactose administered decreases as the infant ages, optionally on a week-to-week, a month-to-month, or a bi-monthly to bi-monthly basis. In certain such embodiments, this decrease in the amount of 3'-sialyllactose administered may be achieved by administration of compositions (e.g., infant formulas) having decreasing concentrations of 3'-sialyllactose. In such embodiments, the concentration of 3'-sialyllactose, on an as-fed basis, may be at least 1500 mg/L through day 5 postpartum, including from 1000 mg/L to 2000 mg/L. In such embodiments, the concentration of 3'-sialyllactose, on an as-fed basis, may be at least 1000 mg/L after day 5 postpartum through day 15 postpartum, including from 500 mg/L to 1500 mg/L. In such embodiments, the concentration of 3'-sialyllactose, on an as-fed basis, may be at least 500 mg/L after day 15 postpartum through 3 months or 6 months postpartum, including from 200 mg/L to 1000 mg/L. Likewise, in certain embodiments of the second, third, and fourth embodiments disclosed herein, the amount of 3'-sialyllactose in the compositions, supplements, and infant formulas administered decreases as the infant ages in the manner as described in the preceding sentences.

In certain embodiments of the first, second, third, and fourth embodiments disclosed herein, the compositions suitable for use in the methods of the present disclosure comprise 3'-sialyllactose at a concentration on an as-fed basis of at least 60 mg/kg bodyweight (of the infant)/day, including from 60 mg/kg bodyweight (of the infant)/day to 250 mg/kg bodyweight (of the infant)/day, also including from 60 mg/kg bodyweight (of the infant)/day to 400 mg/kg bodyweight (of the infant) /day.

In certain embodiments according to the first embodiment, the sialic acid is free sialic acid, and the amount of 3'-sialyllactose administered decreases as the infant ages, optionally on a week-to-week, a month-to-month, or a bi-monthly to bi-monthly basis. In certain such embodiments, the decrease is measured in terms of the total amount of free sialic acid administered per day according to the infant's body weight. In certain such embodiments, the amount of 3'-sialyllactose, on an as-fed basis, may be at least 250 mg/kg bodyweight (of the infant)/day through day 5 postpartum, including from 200 mg/kg bodyweight (of the infant)/day to 400 mg/kg bodyweight (of the infant)/day. In certain such embodiments, the amount of 3'-sialyllactose administered, on an as-fed basis, may be at least 200 mg/kg bodyweight (of the infant)/day after day 5 postpartum through day 15 postpartum, including from 60 mg/kg bodyweight (of the infant) /day to 250 mg/kg bodyweight (of the infant)/day. In certain embodiments of the first embodiment, the amount of 3'-sialyllactose, on an as-fed basis, may be at least 60 mg/kg bodyweight (of the infant)/day after day 15 postpartum through 3 months or 6 months postpartum, including from 60 mg/kg bodyweight (of the infant)/day to 100 mg/kg bodyweight (of the infant)/day. Likewise, in certain embodiments of the second, third, and fourth embodiments disclosed herein, the amount of 3'-sialyllactose in the compositions, nutritional composition, and infant formulas administered decreases as the infant ages in the manner described in the immediately preceding sentences.

Additionally, in certain embodiments of the first, second, third, and fourth embodiments disclosed herein, the total amount of 3'-sialyllactose administered to the infant may be roughly equal to the total amount of sialic acid present in sialylated structures found in normal human breast milk during the first 6 months of lactation, including from 500 mg/L to 1500 mg/L. For the avoidance of any doubt, the amount of sialic acid in "normal" human breast milk, as that term is used herein, is 500 mg/L to 1500 mg/L. In still other embodiments of the first, second, third, and fourth embodiments disclosed herein, the total amount of 3'-sialyllactose administered to the infant is intended to be somewhat less than the total amount present in normal human breast milk during the first 6 months of lactation.

In certain embodiments of the first, second, third, and fourth embodiments disclosed herein, the 3'-sialyllactose may be obtained from and added as a separate ingredient to the infant formula that is administered to an infant, in which case the added 3'-sialyllactose is combined with any inherent sialic acid from other ingredients to provide the total sialic acid content in the infant formula.

As an individual compound or moiety, 3'-sialyllactose has the molecular formula C₂₃H₃₉NO₁₉, a molecular weight of 633.55106 Daltons and is represented by the chemical structure in Equation (2). In 3'-sialyllactose, the N-acetylneuraminyl (NeuAc) unit is connected to the galactosyl unit of lactose at the 3 position. 3'sialyllactose is commonly abbreviated as 3'-SL and is also known by other names such as 2>3')-a-Sialyllactose, 3'-(N-Acetyl-A-neuraminosyl)lactose, 3'-a-Sialyllactose, 3'-Monosialyllactose, 3'-N-Acetylneuraminyl-D-lactose, and 3'-Sialyl-D-lactose.

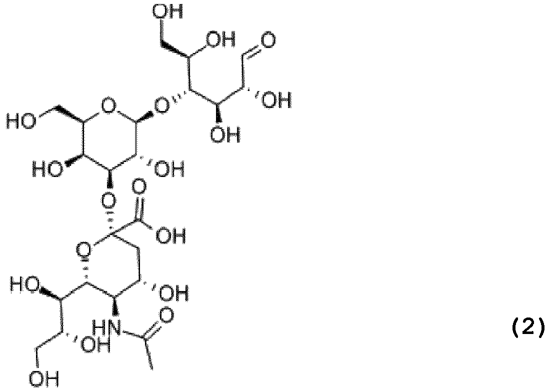

In certain embodiments of the methods according to the first, second, third, and fourth embodiments disclosed herein, the 3'-sialyllactose is administered in nutritional compositions in combination with other components, e.g., prebiotic oligosaccharides, probiotics, etc. In certain embodiments of the methods of the first, second, third, and fourth embodiments disclosed herein, the 3'-sialyllactose is included in a nutritional composition with multiple additional components. Suitable 3'-sialyllactose for use in the methods of the first, second, third, and fourth embodiments disclosed herein may be produced via fermentation, chemical synthesis, or combinations thereof.

### 6'-sialyllactosamine

In certain embodiments of the methods of the first, second, third, and fourth embodiments disclosed herein, the sialic acid that is administered is 6'-sialyllactosamine. In certain such embodiments, compositions suitable for use in the methods may comprise 6'-sialyllactosamine at a concentration on an as-fed basis of at least 500 mg/L, including from 500 mg/L to 1500 mg/L, also including from 500 mg/L to 2000 mg/L. It should be understood that the specific amounts of 6'-sialyllactosamine discussed in this paragraph and the following paragraphs of this section (i.e., both the concentrations in terms of mg/L and the amounts administered in terms of mg/kg body weight) are intended to provide examples of the effective amount of 6'-sialyllactosamine that may be administered according to the methods of the first, second, third, and fourth embodiments disclosed herein.

In certain embodiments according to methods of the first embodiment, the sialic acid is 6'-sialyllactosamine and the amount of 6'-sialyllactosamine administered decreases as the infant ages, optionally on a week-to-week, a month-to-month, or a bi-monthly to bi-monthly basis. In certain such embodiments, this decrease in the amount of 6'-sialyllactosamine administered may be achieved by administration of compositions (e.g., infant formulas) having decreasing concentrations of 6'-sialyllactosamine. In such embodiments, the concentration of 6'-sialyllactosamine, on an as-fed basis, may be at least 1500 mg/L through day 5 postpartum, including from 1000 mg/L to 2000 mg/L. In such embodiments, the concentration of 6'-sialyllactosamine, on an as-fed basis, may be at least 1000 mg/L after day 5 postpartum through day 15 postpartum, including from 500 mg/L to 1500 mg/L. In such embodiments, the concentration of 6'-sialyllactosamine, on an as-fed basis, may be at least 500 mg/L after day 15 postpartum through 3 months or 6 months postpartum, including from 200 mg/L to 1000 mg/L. Likewise, in certain embodiments of the second, third, and fourth embodiments disclosed herein, the amount of 6'-sialyllactosamine in the compositions, supplements, and infant formulas administered decreases as the infant ages in the manner as described in the preceding sentences.

In certain embodiments of the first, second, third, and fourth embodiments disclosed herein, the compositions suitable for use in the methods of the present disclosure comprise 6'-sialyllactosamine at a concentration on an as-fed basis of at least 60 mg/kg bodyweight (of the infant)/day, including from 60 mg/kg bodyweight (of the infant)/day, to 250 mg/kg bodyweight (of the infant)/day, also including from 60 mg/kg bodyweight (of the infant)/day to 400 mg/kg bodyweight (of the infant) /day.

In certain embodiments according to the first embodiment, the sialic acid is 6'-sialyllactosamine, and the amount of 6'-sialyllactosamine administered decreases as the infant ages, on a week-to-week, a month-to-month, or a bi-monthly to bi-monthly basis. In certain such embodiments, the decrease is measured in terms of the total amount of free sialic acid administered per day according to the infant's body weight. In certain such embodiments, the amount of 6'-sialyllactosamine, on an as-fed basis, may be at least 250 mg/kg bodyweight (of the infant)/day through day 5 postpartum, including from 200 mg/kg bodyweight (of the infant)/day to 400 mg/kg bodyweight (of the infant)/day. In certain such embodiments, the amount of 6'-sialyllactosamine administered, on an as-fed basis, may be at least 200 mg/kg bodyweight (of the infant)/day after day 5 postpartum through day 15 postpartum, including from 60 mg/kg bodyweight (of the infant)/day to 250 mg/kg bodyweight (of the infant)/day. In certain embodiments of the first embodiment, the amount of 6'-sialyllactosamine, on an as-fed basis, may be at least 60 mg/kg bodyweight (of the infant)/day after day 15 postpartum through 3 months or 6 months postpartum, including from 60 mg/kg bodyweight (of the infant)/day to 100 mg/kg bodyweight (of the infant)/day. Likewise, in certain embodiments of the second, third, and fourth embodiments disclosed herein, the amount of 6'-sialyllactosamine in the compositions, nutritional composition, and infant formulas administered decreases as the infant ages in the manner described in the immediately preceding sentences.

Additionally, in certain embodiments of the first, second, third, and fourth embodiments disclosed herein, the total amount of 6'-sialyllactosamine administered to the infant may be roughly equal to the total amount of sialic acid present in sialylated structures found in normal human breast milk during the first 6 months of lactation, including from 500 mg/L to 1500 mg/L. For the avoidance of any doubt, the amount of sialic acid in "normal" human breast milk, as that term is used herein, is 500 mg/L to 1500 mg/L. In still other embodiments of the first, second, third, and fourth embodiments disclosed herein, the total amount of 6'-sialyllactosamine administered to the infant is intended to be somewhat less than the total amount present in normal human breast milk during the first 6 months of lactation.

In certain embodiments of the first, second, third, and fourth embodiments disclosed herein, the 6'-sialyllactosamine may be obtained from and added as a separate ingredient to the infant formula that is administered to an infant, in which case the added 6'-sialyllactosamine is combined with any inherent sialic acid from other ingredients to provide the total sialic acid content in the infant formula.

In certain embodiments of the methods according to the first, second, third, and fourth embodiments disclosed herein, the 6'-sialyllactosamine is administered in nutritional compositions in combination with other components, e.g., prebiotic oligosaccharides, probiotics, etc. In certain embodiments of the methods of the first, second, third, and fourth embodiments disclosed herein, the 6'-sialyllactosamine is included in a nutritional composition with multiple additional components. Suitable 6'-sialyllactosamine for use in the methods of the first, second, third, and fourth embodiments disclosed herein may be produced via fermentation, chemical synthesis, or combinations thereof.

### 3'-sialyllactosamine

In certain embodiments of the methods of the first, second, third, and fourth embodiments disclosed herein, the sialic acid that is administered is 3'-sialyllactosamine. In certain such embodiments, compositions suitable for use in the methods may comprise 3'-sialyllactosamine at a concentration on an as-fed basis of at least 500 mg/L, including from 500 mg/L to 1500 mg/L, also including from 500 mg/L to 2000 mg/L. It should be understood that the specific amounts of 3'-sialyllactosamine discussed in this paragraph and the following paragraphs of this section (i.e., both the concentrations in terms of mg/L and the amounts administered in terms of mg/kg body weight) are intended to provide examples of the effective amount of 3'-sialyllactosamine that may be administered according to the methods of the first, second, third, and fourth embodiments disclosed herein.

In certain embodiments according to methods of the first embodiment, the sialic acid is 3'-sialyllactosamine and the amount of 3'-sialyllactosamine administered decreases as the infant ages, optionally on a week-to-week, a month-to-month, or a bi-monthly to bi-monthly basis. In certain such embodiments, this decrease in the amount of 3'-sialyllactosamine administered may be achieved by administration of compositions (e.g., infant formulas) having decreasing concentrations of 3'-sialyllactosamine. In such embodiments, the concentration of 3'-sialyllactosamine, on an as-fed basis, may be at least 1500 mg/L through day 5 postpartum, including from 1000 mg/L to 2000 mg/L. In such embodiments, the concentration of 3'-sialyllactosamine, on an as-fed basis, may be at least 1000 mg/L after day 5 postpartum through day 15 postpartum, including from 500 mg/L to 1500 mg/L. In such embodiments, the concentration of 3'-sialyllactosamine, on an as-fed basis, may be at least 500 mg/L after day 15 postpartum through 3 months or 6 months postpartum, including from 200 mg/L to 1000 mg/L. Likewise, in certain embodiments of the second, third, and fourth embodiments disclosed herein, the amount of 3'-sialyllactosamine in the compositions, supplements, and infant formulas administered decreases as the infant ages in the manner as described in the preceding sentences.

In certain embodiments of the first, second, third, and fourth embodiments disclosed herein, the compositions suitable for use in the methods of the present disclosure comprise 3'-sialyllactosamine at a concentration on an as-fed basis of at least 60 mg/kg bodyweight (of the infant)/day, including from 60 mg/kg bodyweight (of the infant)/day to 250 mg/kg bodyweight (of the infant)/day, also including from 60 mg/kg bodyweight (of the infant)/day to 400 mg/kg bodyweight (of the infant) /day.

In certain embodiments according to the first embodiment, the sialic acid is 3'-sialyllactosamine, and the amount of 3'-sialyllactosamine administered decreases as the infant ages, optionally on a week-to-week, a month-to-month, or a bi-monthly to bi-monthly basis. In certain such embodiments, the decrease is measured in terms of the total amount of free sialic acid administered per day according to the infant's body weight. In certain such embodiments, the amount of 3'-sialyllactosamine, on an as-fed basis, may be at least 250 mg/kg bodyweight (of the infant)/day through day 5 postpartum, including from 200 mg/kg bodyweight (of the infant)/day to 400 mg/kg bodyweight (of the infant)/day. In certain such embodiments, the amount of 3'-sialyllactosamine administered, on an as-fed basis, may be at least 200 mg/kg bodyweight (of the infant)/day after day 5 postpartum through day 15 postpartum, including from 60 mg/kg bodyweight (of the infant)/day to 250 mg/kg bodyweight (of the infant)/day. In certain embodiments of the first embodiment, the amount of 3'-sialyllactosamine, on an as-fed basis, may be at least 60 mg/kg bodyweight (of the infant)/day after day 15 postpartum through 3 months or 6 months postpartum, including from 60 mg/kg bodyweight (of the infant)/day to 100 mg/kg bodyweight (of the infant)/day. Likewise, in certain embodiments of the second, third, and fourth embodiments disclosed herein, the amount of 3'-sialyllactosamine in the compositions, nutritional composition, and infant formulas administered decreases as the infant ages in the manner described in the immediately preceding sentences.

Additionally, in certain embodiments of the first, second, third, and fourth embodiments disclosed herein, the total amount of 3'-sialyllactosamine administered to the infant may be roughly equal to the total amount of sialic acid present in sialylated structures found in normal human breast milk during the first 6 months of lactation, including from 500 mg/L to 1500 mg/L. For the avoidance of any doubt, the amount of sialic acid in "normal" human breast milk, as that term is used herein, is 500 mg/L to 1500 mg/L. In still other embodiments of the first, second, third, and fourth embodiments disclosed herein, the total amount of 3'-sialyllactosamine administered to the infant is intended to be somewhat less than the total amount present in normal human breast milk during the first 6 months of lactation.

In certain embodiments of the first, second, third, and fourth embodiments disclosed herein, the 3'-sialyllactosamine may be obtained from and added as a separate ingredient to the infant formula that is administered to an infant, in which case the added 3'-sialyllactosamine is combined with any inherent sialic acid from other ingredients to provide the total sialic acid content in the infant formula.

In certain embodiments of the methods according to the first, second, third, and fourth embodiments disclosed herein, the 3'-sialyllactosamine is administered in nutritional compositions in combination with other components, e.g., prebiotic oligosaccharides, probiotics, etc. In certain embodiments of the methods of the first, second, third, and fourth embodiments disclosed herein, the 3'-sialyllactosamine is included in a nutritional composition with multiple additional components. Suitable 3'-sialyllactosamine for use in the methods of the first, second, third, and fourth embodiments disclosed herein may be produced via fermentation, chemical synthesis, or combinations thereof.

### Macronutrients

Compositions suitable for use in certain embodiments of the methods of the first, second, third, and fourth embodiments disclosed herein may be formulated to include at least one of protein, fat, and carbohydrate, vitamins, minerals and other nutrients helpful for optimal infant growth and development. In certain such embodiments, the composition is a infant formula containing protein, fat, carbohydrates, minerals, and vitamins.

Although total concentrations or amounts of the fat, protein, and carbohydrates may vary depending upon the product type (i.e., human milk fortifier, preterm infant formula, infant formula, etc.), product form (i.e., nutritional solid, powder, ready-to-feed liquid, or concentrated liquid), and targeted dietary needs of the intended consumer (i.e., the infant), such concentrations or amounts most typically fall within one of the following embodied ranges, inclusive of any other essential fat, protein, and/or carbohydrate ingredients as described herein.

Liquid preterm and term infant formulas suitable for use in administering the sialic acid according to the methods of the first second, third and fourth embodiments, have a carbohydrate concentrations most typically in an amount of from 5% to 40%, including from 7% to 30%, including from 10% to 25%, by weight of the preterm or term infant formula; fat concentrations most typically in an amount from 1% to 30%, including from 2% to 15%, and also including from 3% to 10%, by weight of the preterm or term infant formula; and protein concentrations most typically in an amount from 0.5% to 30%, including from 1% to 15%, and also including from 2% to 10%, by weight of the preterm or term infant formula.

Liquid human milk fortifiers suitable for use in administering the sialic acid according to the methods of the first second, third and fourth embodiments, have a carbohydrate concentration most typically in an amount from 10% to 75%, including from 10% to 50%, including from 20% to 40%, by weight of the human milk fortifier; fat concentrations most typically in an amount from 10% to 40%, including from 15% to 37%, and also including from 18% to 30%, by weight of the human milk fortifier; and protein concentrations most typically in an amount from 5% to 40%, including from 10% to 30%, and also including from 15% to 25%, by weight of the human milk fortifier.

The amount of carbohydrates, fats, and/or proteins in any of the nutritional compositions (e.g., infant formulas) described herein may also be characterized in addition to, or in the alternative, as a percentage of total calories in the liquid nutritional composition as set forth in Table 1.0. These macronutrients for liquid nutritional compositions of the present disclosure are most typically formulated within any of the caloric ranges (embodiments A-F) described in Table 1.0.

**Table 1.0**

| **% of Total Calories** | **Embodiment A** | **Embodiment B** | **Embodiment C** |
|---|---|---|---|
| **Carbohydrate** | 0-98 | 2-96 | 10-75 |
| **Protein** | 0-98 | 2-96 | 5-70 |
| **Fat** | 0-98 | 2-96 | 20-85 |

| | **Embodiment D** | **Embodiment E** | **Embodiment F** |
|---|---|---|---|
| **Carbohydrate** | 30-50 | 25-50 | 25-50 |
| **Protein** | 15-35 | 10-30 | 5-30 |
| **Fat** | 35-55 | 1-20 | 2-20 |

In one specific example according to the methods of the first second, third and fourth embodiments disclosed herein, infant formulas (ready-to-feed liquid, concentrated liquids, and powders) include those embodiments in which the protein component may comprise from 7.5% to 25% of the caloric content of the formula; the carbohydrate component may comprise from 35% to 50% of the total caloric content of the infant formula; and the fat component may comprise from 30% to 60% of the total caloric content of the infant formula. These ranges are provided as examples only, and are not intended to be limiting. Additional suitable ranges are noted in the Table 1.1.

**Table 1.1**

| **% of Total Calories** | **Embodiment G** | **Embodiment H** | **Embodiment I** |
|---|---|---|---|
| **Carbohydrate** | 20-85 | 30-60 | 35-55 |
| **Protein** | 5-70 | 20-60 | 25-50 |
| **Fat** | 2-75 | 5-50 | 7-40 |

When the compositions, or nutritional compositions disclosed herein are powdered preterm or term infant formulas according to certain embodiments of the first second, third and fourth embodiments, the protein component is present in an amount of from 5% to 35%, including from 8% to 12%, and including from 10% to 12% by weight of the preterm or term infant formula; the fat component is present in an amount of from 10% to 35%, including from 25% to 30%, and including from 26% to 28% by weight of the preterm or term infant formula; and the carbohydrate component is present in an amount of from 30% to 85%, including from 45% to 60%, including from 50% to 55% by weight of the preterm or term infant formula.

For powdered human milk fortifiers according to certain embodiments of the first second, third and fourth embodiments, the protein component is present in an amount of from 1% to 55%, including from 10% to 50%, and including from 10% to 30% by weight of the human milk fortifier; the fat component is present in an amount of from 1% to 30%, including from 1% to 25%, and including from 1% to 20% by weight of the human milk fortifier; and the carbohydrate component is present in an amount of from 15% to 75%, including from 15% to 60%, including from 20% to 50% by weight of the human milk fortifier.

The total amount or concentration of fat, carbohydrate, and protein, in the powdered nutritional compositions (e.g., infant formulas) suitable for use in certain embodiments of the methods according to the first, second, third, and fourth embodiments of the present disclosure can vary considerably depending upon the selected composition and dietary or medical needs of the intended user. Additional suitable examples of macronutrient concentrations are set forth below. In this context, the total amount or concentration refers to all fat, carbohydrate, and protein sources in the powdered composition. For powdered nutritional compositions (e.g., infant formulas), such total amounts or concentrations are most typically and preferably formulated within any of the embodied ranges described in the Table 1.2.

**Table 1.2**

| **% Total Calories** | **Embodiment J** | **Embodiment K** | **Embodiment L** |
|---|---|---|---|
| **Carbohydrate** | 1-85 | 30-60 | 35-55 |
| **Fat** | 5-70 | 20-60 | 25-50 |
| **Protein** | 2-75 | 5-50 | 7-40 |

### Fat

The compositions (e.g., infant formulas) suitable for use in the methods according to certain embodiments of the first, second, third and fourth embodiments of the present disclosure may optionally comprise a source or sources of fat. Suitable sources of fat for use herein include any fat or fat source that is suitable for use in an oral nutritional composition (e.g., infant formula) and is compatible with the essential elements and features of such composition. For example, in one specific embodiment, the fat is derived, at least in part, from long chain polyunsaturated fatty acids (LCPUFAs).

Exemplary LCPUFAs for use in the certain compositions (e.g., infant formulas) suitable for use in the methods of the first, second, third and fourth embodiments include, for example, ω-3 LCPUFAs and ω-6 LCPUFAs. Specific LCPUFAs include docosahexaenoic acid (DHA), eicosapentaenoic acid (EPA), docosapentaenoic acid (DPA), arachidonic acid (ARA), linoleic acid, linolenic acid (alpha linolenic acid) and gamma-linolenic acid derived from oil sources such as plant oils, marine plankton, fungal oils, and fish oils. In one particular embodiment, the LCPUFAs are derived from fish oils such as menhaden, salmon, anchovy, cod, halibut, tuna, or herring oil. Particularly preferred LCPUFAs for use in the nutritional compositions (e.g., infant formulas) with the HMOs include DHA, ARA, EPA, DPA, and combinations thereof.

In order to reduce potential side effects of high dosages of LCPUFAs in the compositions, infant formulas, and nutritional compositions, the content of LCPUFAs preferably does not exceed 3% by weight of the total fat content, including below 2% by weight of the total fat content, and including below 1% by weight of the total fat content in the nutritional composition.

The LCPUFA may be provided as free fatty acids, in triglyceride form, in diglyceride form, in monoglyceride form, in phospholipid form, in esterfied form or as a mixture of one or more of the above, preferably in triglyceride form. In another specific embodiment, the fat is derived from short chain fatty acids.

Additional non-limiting examples of suitable fats or sources thereof for use in the nutritional compositions in certain embodiments of the first, second, third and fourth embodiments described herein include coconut oil, fractionated coconut oil, soybean oil, corn oil, olive oil, safflower oil, high oleic safflower oil, oleic acids (EMERSOL 6313 OLEIC ACID, Cognis Oleochemicals, Malaysia), MCT oil (medium chain triglycerides), sunflower oil, high oleic sunflower oil, palm and palm kernel oils, palm olein, canola oil, marine oils, fish oils, fungal oils, algae oils, cottonseed oils, and combinations thereof.

### Protein

The compositions (e.g., infant formulas) suitable for use in certain embodiments of the methods of the first, second, third and fourth embodiments, may optionally further comprise protein. Any protein source that is suitable for use in oral nutritional compositions (e.g., infant formulas) and is compatible with the essential elements and features of such compositions may be utilized. Non-limiting examples of suitable proteins or sources thereof for use in the nutritional compositions (e.g., infant formulas) include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy) or combinations thereof. Non-limiting examples of such proteins include milk protein isolates, milk protein concentrates as described herein, casein protein isolates, extensively hydrolyzed casein, whey protein, sodium or calcium caseinates, whole cow milk, partially or completely defatted milk, soy protein isolates, and soy protein concentrates. In one specific embodiment, the nutritional compositions (e.g., infant formulas) include a protein source derived from milk proteins of bovine origin. In another specific embodiment, the nutritional compositions (e.g., infant formulas) include a protein source derived from milk proteins of human origin.

In certain embodiments according to the methods of the first, second, third, and fourth embodiments disclosed herein, the composition for administering the sialic acid includes a protein source in the form of a hydrolyzed protein hydrolysate. In this context, the terms "hydrolyzed protein" or "protein hydrolysates" are used interchangeably herein and include extensively hydrolyzed proteins, wherein the degree of hydrolysis is most often at least about 20%, including from about 20% to about 80%, and also including from about 30% to about 80%, even more preferably from about 40% to about 60%. The degree of hydrolysis is the extent to which peptide bonds are broken by a hydrolysis method. The degree of protein hydrolysis for purposes of characterizing the extensively hydrolyzed protein component of these embodiments is easily determined by one of ordinary skill in the formulation arts by quantifying the amino nitrogen to total nitrogen ratio (AN/TN) of the protein component of the selected liquid formulation. The amino nitrogen component is quantified by USP titration methods for determining amino nitrogen content, while the total nitrogen component is determined by the Tecator Kjeldahl method, all of which are well known methods to one of ordinary skill in the analytical chemistry art.

Suitable hydrolyzed proteins for use in compositions suitable for use in certain embodiments of the methods of the first, second, third and fourth embodiments, may include soy protein hydrolysate, casein protein hydrolysate, whey protein hydrolysate, rice protein hydrolysate, potato protein hydrolysate, fish protein hydrolysate, egg albumen hydrolysate, gelatin protein hydrolysate, combinations of animal and vegetable protein hydrolysates, and combinations thereof. Particularly preferred protein hydrolysates include whey protein hydrolysate and hydrolyzed sodium caseinate.

In certain embodiments of the methods of the first, second, third and fourth embodiments, the protein source for the composition, infant formula or nutritional composition that is administered may include at least 20% (by weight total protein) protein hydrolysate, including from 30% to 100% (by weight total protein) protein hydrolysate, and including from 40% to 80% (by weight total protein) protein hydrolysate, and including 50% (by weight total protein) protein hydrolysate. In one particular embodiment according to the methods of the first, second, third, and fourth embodiments disclosed herein, the nutritional composition that is administered includes 100% (by weight total protein) protein hydrolysate.

### Carbohydrate

Compositions suitable for use in the methods of the present disclosure may further optionally comprise one or more carbohydrates that are suitable for use in an oral nutritional composition and are compatible with the essential elements and features of such compositions.

Non-limiting examples of suitable carbohydrates or sources thereof for use in the nutritional compositions suitable for use in the methods of certain embodiments of the first, second, third, and fourth embodiments described herein may include maltodextrin, hydrolyzed or modified starch or cornstarch, glucose polymers, corn syrup, corn syrup solids, rice-derived carbohydrates, pea-derived carbohydrates, potato-derived carbohydrates, tapioca, sucrose, glucose, fructose, lactose, high fructose corn syrup, honey, sugar alcohols (e.g., maltitol, erythritol, sorbitol), artificial sweeteners (e.g., sucralose, acesulfame potassium, stevia) and combinations thereof. A particularly desirable carbohydrate is a low dextrose equivalent (DE) maltodextrin.

Certain embodiments of the first, second, third and fourth embodiments, when used in the compositions, supplements, infant formulas, or nutritional compositions, the carbohydrate source may comprise at least 25 weight % of the total composition, including from 0 to 98 weight % of the total composition, including from 30 to 50 weight % of the total composition, including from 20 to 85 weight % of the total composition, including from 1 to 85 weight % of the total composition, and including 50 weight % of the total composition.

### Other Optional Ingredients

Compositions (e.g., infant formulas) suitable for use in certain embodiments of the methods of the first, second, third and fourth embodiments, may further comprise other optional components that may modify the physical, chemical, aesthetic or processing characteristics of the compositions or serve as pharmaceutical or additional nutritional components when used in the targeted population. Many such optional ingredients that are known or otherwise suitable for use in medical food or other nutritional products or pharmaceutical dosage forms and may also be used in the compositions herein, provided that such optional ingredients are safe for oral administration and are compatible with the essential and other ingredients in the selected product form.

Non-limiting examples of such optional ingredients include preservatives, emulsifying agents, buffers, pharmaceutical actives, anti-inflammatory agents, additional nutrients as described herein, colorants, flavors, thickening agents and stabilizers, emulsifying agents, lubricants, and so forth.

Compositions (e.g., infant formulas) suitable for use in certain embodiments of the methods of the first, second, third and fourth embodiments, may further comprise a sweetening agent, preferably including at least one sugar alcohol such as maltitol, erythritol, sorbitol, xylitol, mannitol, isolmalt, and lactitol, and also preferably including at least one artificial or high potency sweetener such as acesulfame K, aspartame, sucralose, saccharin, stevia, and tagatose. These sweetening agents, especially as a combination of a sugar alcohol and an artificial sweetener, are especially useful in formulating liquid beverage embodiments of the present disclosure having a desirable favor profile. These sweetener combinations are especially effective in masking undesirable flavors sometimes associated with the addition of vegetable proteins to a liquid beverage. Optional sugar alcohol concentrations in the nutritional composition may range from at least 0.01%, including from 0.1% to about 10%, and also including from about 1% to about 6%, by weight of the nutritional composition. Optional artificial sweetener concentrations may range from about 0.01%, including from about 0.05% to about 5%, also including from about 0.1 % to about 1.0%, by weight of the nutritional composition.

A flowing agent or anti-caking agent may be included in the compositions (e.g., infant formulas) suitable for use in certain embodiments of the methods according to the first, second, third, and fourth embodiments described herein to retard clumping or caking of the powder over time and to make a powder embodiment flow easily from its container. Any known flowing or anti-caking agents that are known or otherwise suitable for use in a nutritional powder or product form are suitable for use herein, non-limiting examples of which include tricalcium phosphate, silicates, and combinations thereof. The concentration of the flowing agent or anti-caking agent in the nutritional composition (e.g., infant formula) varies depending upon the product form, the other selected ingredients, the desired flow properties, and so forth, but most typically range from about 0.1% to about 4%, including from about 0.5% to about 2%, by weight of the nutritional composition.

A stabilizer may also be included in the compositions, infant formulas, and nutritional compositions suitable for use in certain embodiments of the methods according to the first, second, third, and fourth embodiments disclosed herein. Any stabilizer that is known or otherwise suitable for use in a nutritional composition is also suitable for use herein, some non-limiting examples of which include gums such as xanthan gum. The stabilizer may represent from about 0.1% to about 5.0%, including from about 0.5% to about 3%, including from about 0.7%> to about 1.5%, by weight of the nutritional composition (e.g., infant formula).

Additionally, the compositions (e.g., infant formulas) suitable for use in the certain embodiments of the methods according to the first, second, third, and fourth embodiments described herein may comprise one or more antioxidants to provide nutritional support, as well as to reduce oxidative stress. Any antioxidants suitable for oral administration may be included for in the compositions suitable for use in the methods of the present disclosure, including, for example, vitamin A, vitamin E, vitamin C, retinol, tocopherol, and carotenoids.

In one specific embodiment according to the methods of the first, second, third, and fourth embodiments disclosed herein, the antioxidants for use in the compositions (e.g., infant formulas) include carotenoids such as lutein, zeaxanthin, lycopene, beta-carotene, and combinations thereof, and particularly, combinations of the carotenoids lutein, lycopene, and beta-carotene. Compositions containing these combinations, as selected and defined herein, can be used to modulate inflammation and/or levels of C-reactive protein in preterm and term infants.

The compositions (e.g., infant formulas) for use in certain embodiments of the methods according to the first, second, third, and fourth embodiments disclosed herein may further comprise any of a variety of other vitamins or related nutrients, non-limiting examples of which include vitamin D, vitamin K, thiamine, riboflavin, pyridoxine, vitamin B₁₂, niacin, folic acid, pantothenic acid, biotin, choline, inositol, salts and derivatives thereof, and combinations thereof.

The compositions (e.g., infant formulas) for use in certain embodiments of the methods according to the first, second, third, and fourth embodiments disclosed herein may further comprise any of a variety of other additional minerals, non-limiting examples of which include calcium, phosphorus, magnesium, iron, zinc, manganese, copper, sodium, potassium, molybdenum, chromium, chloride, and combinations thereof.

The compositions (e.g., infant formulas) for use in certain embodiments of the methods according to the first, second, third, and fourth embodiments disclosed herein may additionally comprise nucleotides and/or nucleotide precursors selected from the group consisting of nucleoside, purine base, pyrimidine base, ribose and deoxyribose. The nucleotide may be in monophosphate, diphosphate, or triphosphate form. The nucleotide may be a ribonucleotide or a deoxyribonucleotide. The nucleotides may be monomeric, dimeric, or polymeric (including R A and DNA). The nucleotide may be present in the nutritional composition as a free acid or in the form of a salt, preferably a monosodium salt.

Suitable nucleotides and/or nucleosides for use in compositions (e.g., infant formulas) for certain embodiments of the methods according to the first, second, third, and fourth embodiments disclosed herein may include one or more of cytidine 5'-monophosphate, uridine 5'-monophosphate, adenosine 5'-monophosphate, guanosine 5'-1-monophosphate, and/or inosine 5'-monophosphate, more preferably cytidine 5'-monophosphate, uridine 5'-monophosphate, adenosine 5'-monophosphate, guanosine 5'-monophosphate, and inosine 5'-monophosphate.

### Methods of Manufacture

Compositions suitable for use in the certain embodiments according to the methods of the first, second, third, and fourth embodiments of the present disclosure may be prepared by any known or otherwise effective manufacturing technique for preparing the selected product solid or liquid form. Many such techniques are known for any given product form such as nutritional liquids or powders and can easily be applied by one of ordinary skill in the art to the compositions suitable for use in the methods described herein.

Compositions suitable for use in certain embodiments according to the methods of the first, second, third, and fourth embodiments of the present disclosure can therefore be prepared by any of a variety of known or otherwise effective formulation or manufacturing methods. In one suitable manufacturing process, for example, at least three separate slurries are prepared, including a protein-in-fat (PIF) slurry, a carbohydrate-mineral (CHO- MIN) slurry, and a protein-in-water (PIW) slurry. The PIF slurry is formed by heating and mixing the oil (e.g., canola oil, corn oil, etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents (e.g. avicel, gellan, and carrageenan). The resulting CHO-MIN slurry is held for 10 minutes with continued heat and agitation before adding additional minerals (e.g., potassium chloride, magnesium carbonate, potassium iodide, etc.), and/or carbohydrates (e.g., HMOs, fructooligosaccharide, sucrose, corn syrup, etc.). The PIW slurry is then formed by mixing with heat and agitation the remaining protein, if any.

The resulting slurries are then blended together with heated agitation and the pH adjusted to 6.6-7.0, after which the composition is subjected to high-temperature short-time (HTST) processing during which the composition is heat treated, emulsified and homogenized, and then allowed to cool. Water soluble vitamins and ascorbic acid are added, the pH is adjusted to the desired range if necessary, flavors are added, and water is added to achieve the desired total solid level. The composition is then aseptically packaged to form an aseptically packaged nutritional emulsion. This emulsion can then be further diluted, heat-treated, and packaged to form a ready-to-feed or concentrated liquid, or it can be heat-treated and subsequently processed and packaged as a reconstitutable powder, e.g., spray dried, drymixed, agglomerated.

The nutritional solid, such as a spray dried nutritional powder or drymixed nutritional powder, may be prepared by any collection of known or otherwise effective techniques, suitable for making and formulating a nutritional powder.

For example, when the nutritional powder is a spray dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried nutritional powders herein.

One method of preparing the spray dried nutritional powder comprises forming and homogenizing an aqueous slurry or liquid comprising predigested fat, and optionally protein, carbohydrate, and other sources of fat, and then spray drying the slurry or liquid to produce a spray dried nutritional powder. The method may further comprise the step of spray drying, drymixing, or otherwise adding additional nutritional ingredients, including any one or more of the ingredients described herein, to the spray dried nutritional powder.

### Methods of Use

The methods according to the first, second, third, and fourth embodiments described herein are primarily for use with formula or bottle fed infants. Formula or bottle fed infants can, in certain embodiments, be considered to include infants that are breast fed but supplemented with infant formula. Such formula fed or bottle fed infants are typically not suffering from any particular disease, disorder, or condition. Although, it should be understood that formula fed or bottle fed infants that have been diagnosed with one or more diseases, disorders or conditions, could benefit from the methods disclosed herein. In certain embodiments, the methods according to the first, second, third, and fourth embodiments described herein may be particularly useful with preterm infants. Generally, the compositions, nutritional compositions and infant formulas administered according to the methods of the first, second, third, and fourth embodiments described herein are intended to simulate certain aspects of human breast milk; in other words, to provide certain benefits of human breast milk to bottle fed or formula fed infants who otherwise would be receiving lower levels of sialic acid than an infant that is breast fed. In certain embodiments according to the methods of the first, second, third, and fourth embodiments, the infant may be a breast fed infant that is not receiving sufficient sialic acid from the breast milk that it is ingesting. In certain embodiments, the infant administered the sialic acid according to the methods of the first, second, third, and fourth embodiments disclosed herein may have experienced a reduction in memory, learning or cognitive processing or alternatively may only be at increased risk of experiencing reduction in memory, learning or cognitive processing (susceptible to experiencing reduction in memory, learning or cognitive processing) due to family history or other medical conditions, for example; preterm infants can be considered to one such subset of infants that can fall with each of the foregoing categories.

In certain embodiments of the first, second, third, and fourth embodiments disclosed herein, the methods of the present disclosure are used to administer sialic acid to a healthy infant for a time period after delivery, (i.e., after birth) and optionally subsequent to the infant being weaned to provide the infant with sialic acid after delivery. The infant receiving the sialic acid via the compositions, nutritional compositions, and infant formulas according to the methods of the first, second, third, and fourth embodiments disclosed herein may have no family history of memory, learning or cognitive developmental problems, or alternatively may be an infant with a family history of or predisposition towards memory, learning or cognitive developmental problems, such that the infant may be considered to be susceptible to (or at risk of) developing memory, learning or cognitive developmental issues/problems

The first embodiment disclosed herein is directed to methods of achieving sustained memory improvement in an infant. The method comprises administering an effective amount of sialic acid to the infant until at least 90 days after birth. According to the first embodiment, the amount of sialic acid administered decreases as the infant ages, optionally on a week-to-week basis, a month-to-month basis, or a bi-monthly to bi-monthly basis. Further, in the first embodiment, the administration results in enhanced brain function in the infant and sustained memory improvement at least until the infant reaches childhood.

The fourth embodiment disclosed herein is directed to methods of achieving sustained memory improvement in an infant comprising combining a composition having sialic acid with an infant formula that is low in sialoglycoconjugated compounds. In the fourth embodiment the formula is supplemented with sialic acid, either in a free form or as a sialyloligosaccharide, in order to increase the low level of sialic acid present in the infant formula (which may be milk-based formula) and ultimately make it more similar to human breast milk. According to the fourth embodiment, the combination is administered to the infant. Further, in the fourth embodiment, the administration of the combination results in sustained memory improvement at least until the infant reaches childhood.

In certain embodiments of the first, second, third and forth embodiment disclosed herein, an infant that is administered the sialic acid may actually have or be afflicted with, may be susceptible to, may be at risk of contracting, or may be at elevated risk as compared to the general population for contracting, acquiring, or developing the following diseases, disorders, or conditions: Hereditary Inclusion Body Myopathy (HIBM or HIBM type 2), Nonaka myopathy, Distal Myopathy with Rimmed Vacuoles (DMRV), alterations in the immune response, an increased risk of cardiovascular disease, impairment of neuronal sprouting, epilepsy, blindness, or death. These are all clinical diseases or conditions linked to or resulting from a reduction or decrease in sialic acid production. It is noteworthy that the total absence of sialic acid in the brain is lethal and death occurs early in the embryonic development in its absence.

In certain embodiments of the first, second, third, and fourth embodiments disclosed herein, the total amount of sialic acid administered to the infant is equal to (or intended to be approximately equal to) the total amount present in normal human breast milk during the first 3 months of lactation. In other words, the total amount of sialic acid administered to the infant on a day-to-day basis according to the such methods, may be equal to (or approximately equal to) the amount of sialic acid that an infant would receive during the equivalent time period if fed normal human breast milk. Alternatively in certain embodiments, the total amount of sialic acid administered to the infant is equal to the total amount present in normal human breast milk during the first 6 months of lactation. Alternatively in certain embodiments, the amount of sialic acid administered may be greater than the amount of sialic acid present in normal human breast milk after birth and continuing through postnatal day 183 or greater than the total amount present in normal human breast milk during the first 6 months of lactation.

In certain embodiments of the methods according to the first, second, third, and fourth embodiments disclosed herein, the compositions, nutritional compositions, and infant formulas comprising sialic acid may be administered to the infant orally, as needed to provide an effective amount of sialic to provide the memory and learning ability improvements desired. In other embodiments, the sialic acid may be administered to the infant enterally.

In certain embodiments of the methods according to the first, second, third, and fourth embodiments disclosed herein, the compositions, nutritional compositions, and infant formulas comprising the sialic acid are administered directly to the infant prior to or after weaning, i.e., prior to weaning from breast milk, while breast milk is the infant's primary source of feeding, while breast milk is the infant's partial source of feeding, after weaning from breast milk or combinations thereof, to provide sialic acid to the growing and developing infant. In certain embodiments of the methods of the first, second, third, and fourth embodiments disclosed herein, the compositions, supplements, and infant formulas may be administered to the infant from birth up to and after 6 months after birth. In certain embodiments of the methods of the first, second, third, and fourth embodiments disclosed herein, the infant may continue to receive the sialic acid after weaning through 6 months after birth including, one month, two months, three months, four months, five months and six months after birth.

In certain embodiments of the methods of the first, second, third, and fourth embodiments disclosed herein, the sialic acid may be administered to the infant at least once daily, including at least twice daily, including at least three times daily, including at least four times daily, including at least five times daily, including at least six times daily, including at least seven times daily, including at least eight times daily, and including at least nine times daily, including at least ten times daily. In certain such embodiments the sialic acid may be administered 1 to 10 times daily or more, including 2 to 10 times daily, including 3 to 10 times daily, including 4 to 10 times daily, including 5 to 10 times daily, including 6 to 10 times daily, including 7 to 10 times daily, including 8 to 10 times daily, and including 9 to 10 times daily.

In certain embodiments of the first, second, third, and fourth embodiments disclosed herein, where the composition or nutritional composition is provided after weaning the infant, the sialic acid may be in the form of an infant formula, and potentially, a follow-on formula, a pediatric formula, or the like, depending upon the age of the infant. The composition, or human milk fortifier having sialic acid may be administered by combining it with human breast milk, an infant formula, and potentially, a follow-on formula, a pediatric formula, or the like, depending upon the age of the infant.

The composition, nutritional composition or infant formula administered according to the methods of certain embodiments of the first, second, third, and fourth embodiments disclosed herein, may be administered to the infant as a supplement while the infant is still being bottle fed. In certain embodiments of the methods according to the first, second, third, and fourth embodiments disclosed herein, the composition, nutritional composition, or infant formula may be administered orally via a bottle, a syringe, dropper, droplets, or any other administering device or method known in the art.

As previously discussed, according to the methods of the first, second, third and fourth embodiments the administration of an effective amount of sialic acid to premature infants and full term infants results in sustained memory improvement, sustained improvement in learning abilities, or both, through dietary intervention. In certain embodiments, the memory enhancement, learning ability enhancement, or both can be understood as an improvement in memory as compared to the memory without the administration of sialic acid, an improvement in learning abilities as compared to the learning abilities without the administration of sialic acid. In certain embodiments according to the methods of the first, second, and fourth embodiments, the improvement in sustained memory, improvement in learning abilities, or both, is at least a 10% improvement as compared to the memory, learning abilities, or both, respectively, of the individual without the administration of the sialic acid, including a 10% to 50% improvement in memory, learning abilities, or both; a 10% to 30% improvement in memory, learning abilities or both; and at least a 10% to 100% improvement in memory, learning abilities, or both. In certain other embodiments according to the methods of the first, second, and fourth embodiments, the memory improvement, learning abilities improvement, or both, may be seen as at least a 30% to 50% improvement in memory, learning abilities, or both, respectively. In certain embodiments according to the methods of the first, second, third, and fourth embodiments, the effect of the administration is that hippocampus function in the brain of the infant is enhanced by at least 10%, including about 10% to about 100%.

Generally, an improvement in memory and learning abilities, including a sustained improvement can be measured in various ways. In humans, testing and neuroimaging for hippocampus and brain function are well-known in the art and readily available to psychologists, neuroradiologists or neuroscientists. The memory and learning abilities improvement resulting from the methods according to the first, second, third, and fourth embodiments disclosed herein could be measured using any commonly used psychological test, including without limitation, 'Kiel Locomotor Maze', containing features of the Radial Arm Maze and the Morris Water Maze, in order to assess spatial memory and orientation, which has been optimized for school age children, or Cambridge Neuropsychological Test Automated Battery (CANTAB), a computerized battery of nonverbal visually-presented neuropsychological tests, designed to test spatial memory span, spatial working memory and spatial recognition. Additionally, a sustained memory improvement and sustained improvement in learning abilities according to the first, second, third, and fourth embodiments disclosed herein can be shown via comparative testing in animals (e.g., rats or mice), using the same composition used to administered sialic acid to infants.

As previously discussed, according to the methods of the first, second, and fourth embodiments, the administration of an effective amount of sialic acid results in a sustained memory improvement at least until the infant reaches childhood, including through age 2, age 3, age 4, age 5, age 6, age 7, age 8, age 9, and age 10. According to the methods of the third embodiment, the administration of an effective amount of sialic acid results in a sustained improvement in learning abilities at least until the infant reaches childhood, including through age 2, age 3, age 4, age 5, age 6, age 7, age 8, age 9, and age 10. According to certain other embodiments of the methods of the first, second, third and fourth embodiments, the administration of an effective amount of sialic acid results in a sustained memory improvement and/or sustained improvement in learning abilities through adolescence, including through age 10, age 11, age 12, age 13, age 14, age 15, age 16, age 17, and age 18. In certain other embodiments of the first, second, third and fourth embodiments, the administration of an effective amount of sialic acid results in a sustained memory improvement and/or sustained improvement in learning abilities through adulthood, including through age 18, age 19, age 20, age 21 and above. In still other embodiments of the first, second, third and fourth embodiments, the memory improvement and/or sustained improvement in learning abilities is sustained on a long term basis, i.e., until the human is between 10 years to 21 years of age, including between 11 years to 22 years of age, including between 12 to 23 years of age, including between 13 to 24 years of age, including between 15 to 25 years of age, including between 16 to 27 years of age, including between 17 to 28 years of age, and the like.

### EXAMPLES

The following examples illustrate specific embodiments and/or features of the methods of the present disclosure. The examples are given solely for the purpose of illustration and are not to be construed as limitations of the present disclosure, as many variations thereof are possible without departing from the spirit and scope of the disclosure.

These examples illustrate certain embodiments according to the first, second, third, and fourth embodiments disclosed herein. Free sialic acid or 6'-sialyllactose (6'-SL) was administered to Sprague-Dawley rat pups during the first stages of development and the effect of the administration on memory, learning and cognitive abilities later in life was measured and analyzed.

### EXAMPLE 1

In order to establish the role of free sialic acid in cognition, rat pups were supplemented with free N-Acetylneuraminic acid (Neu5Ac) from postnatal day 3 (PND3) and their learning and memory abilities were analyzed later in life. Rat performance in a classical spatial cognitive task (Y-maze) was evaluated once the animals were weaned. LTP response in hippocampus was also evaluated postnatal day 110 (PND110).

Similar to human breast milk, rat milk is very rich in sialylated structures. The sialic acid content in rat milk peaks around lactation day 8. After day 8, the amount of sialylated structures in rat milk begins to decrease and continues to decrease until day 16. From that day to the end of lactation, the level of sialylated compounds remains very low. In order to bypass milk containing the highest levels of sialylated structures, two cohorts of pregnant Sprague-Dawley rats were utilized: the foster mothers in the first cohort and the biological mothers in the second cohort. The first cohort contained 8 mothers. The first cohort gave birth 16 days before the second cohort.

The second cohort contained 12 mothers. Once the pups born to the second cohort were 3 days old, they were removed from their biological mothers and given to foster mothers in the first cohort whose own pups were removed and sacrificed. Each foster mother received 5 male and 5 females 3 days-old pups. Besides, 4 mothers from the second cohort also received 5 male and 5 female 3 days-old pups per mother. Thus, the pups fed by the mothers from the first cohort received milk containing less sialic acid than the pups fed by mothers from the second cohort, which underwent a normal lactation. Being very altricial, some of the pups which were fed the low sialic acid milk died before the end of the lactation, resulting in 28 male pups remaining to complete the testing. No relationship between survival and diet was observed.

The pups from the second cohort were divided into two groups and given the following designations: the SIA-supplemented group and the Control group. Pups in the SIA - supplemented group were given milk containing low sialic acid, and received a supplementation of free sialic acid since the same day of transfer to the foster mothers until weaning. There were a total of 12 male pups in the SIA -supplemented group. The SIA -supplemented group received free sialic acid supplementation (8 doses/day) in order to reach the same free sialic acid levels as normally reared pups. The amount of free sialic acid given to the SIA-supplemented group was selected to follow the change in concentration of sialic acid in rat milk, ranging from 1.5 g/Kg body weight on day 3 to 0.5 g/Kg body weight at weaning. The amount of sialic acid given to any pup was a maximum of 1.8 mg/Kg body weight on day 8.

The Control group of rat pups was fed milk containing low free sialic acid content and received only vehicle as a supplement. The composition of the vehicle was 0.9% NaCl in water. There were 16 male pups in the Control group.

Pups fed by mothers from the second cohort were normally reared since they received normal breast milk from their mothers for the whole lactation period. These pups were designated as the "Reference Group."

All pups in each of the three groups utilized in these examples were male. The pups in each group were weaned after 21 days. Once weaned, all the animals were fed standard chow diet regardless of the experimental group to which they belonged.

### Y-maze testing

The Y-maze is a widely accepted method of testing spatial orientation and memory capabilities in rats. Ten rats in each group were evaluated in the Y-maze. At postnatal day 90 (PND90) for each group, the rats were subjected to a training trial (hereinafter, the "Training Trial"). The approximate equivalent in human years to 90 days of rat life is 10 to 20 years of age. At 5 minutes post training, rats in each group were tested again to test their retention (hereinafter, the "Retention Trial"). At 48 hours post training, rats in each group were tested again to test their memory, (hereinafter, the "Memory Trial").

During the Training Trial, one of the three arms of the Y-maze was blocked, and animals were allowed to explore the other two arms for 15 minutes. During the Retention Trial and the Memory Trial, all three arms were open and animals were allowed to explore for 5 minutes. The amount of time each animal spent at the start of the Y-maze and each arm was evaluated. By spending a longer time visiting the previously blocked arm during the Retention Trial and the Memory Trial, an animal displays a normal interest in novelty and an apparent ability to remember which arms it has previously visited.

### Results of Retention Trial

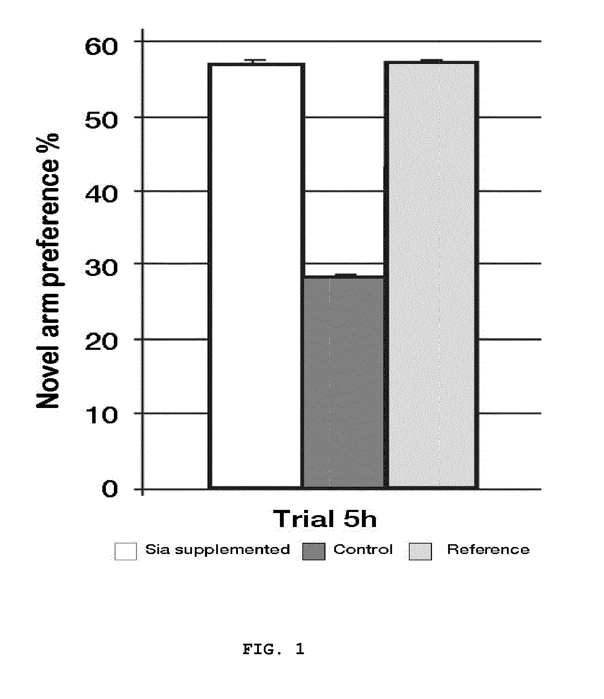

FIG. 1 depicts the results of the Retention Trial for each of the three groups of rats. FIG. 1 depicts the percentage the animals in each group that preferred the novel arm first during the retention test. The result are expressed as mean±SEM. The "*" denotes significant differences.

During the Retention Trial, normal young adult rats in the Reference Group were attracted by novelty and tended to visit the novel arm before any of the two familiar ones. As illustrated in FIG. 1, the Reference Group demonstrated a novel arm preference of about 57%.

Rats in the SIA-supplemented Group performed similar to the Reference Group during the Retention Trial, preferring to visit the novel arm before any of the two familiar ones. Referring again to FIG. 1, the SIA-supplemented Group demonstrated a novel arm preference of about 57%.

Rats in the Control Group demonstrated a lower preference for visiting the novel arm than did rats in any other group. Referring again to FIG. 1, the Control Group demonstrated a novel arm preference of about 28%.

### Results of Memory Trial

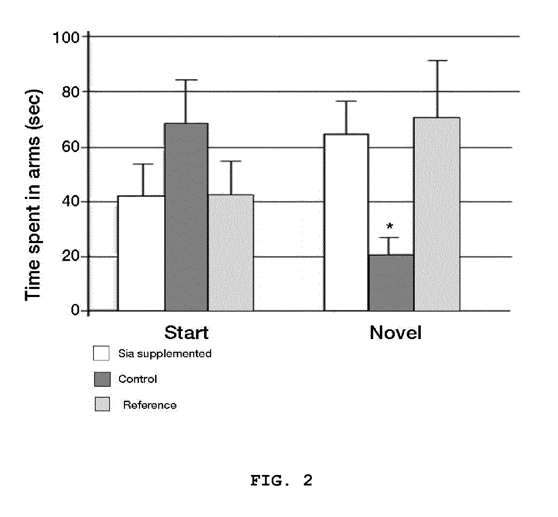

FIG. 2 depicts the results of the Memory Trial. FIG. 2 illustrates the time spent in the start and novel arms during the Memory Trial. Results are expressed as mean±SEM. The "*" denotes significant differences.

During the Memory Trial, performed 48 hours after the Training Trial, a long time spent at the start of the Y-maze denotes anxiety and neophobia. Conversely, the longer time an animal spent at the novel arm indicates an interest in novelty and apparent ability to recognize which arms they had visited previously. To demonstrate this ability the animals must recall which of the three arms was visited only once, i.e., during the Retention Trial, and which ones they visited twice, i.e., during the Training Trial and the Memory Trial.

Rats in the Control Group visited the novel arm less than the familiar arms. Referring to FIG. 2, the Control Group spent about 70 seconds in the start arm, while only spending about 20 seconds in the novel arm. Additionally, the Control Group spent more time in the start arm than in any of the other arms.

Rats in the SIA-supplemented Group spent more of their time in the novel arm. Referring to FIG. 2, the SIA-supplemented Group spent about 40 seconds in the start arm, while spending about 65 seconds in the novel arm.

Rats in the Reference Group spent more of their time in the novel arm. Referring to FIG. 2, the Reference Group spent about 40 seconds in the start arm, while spending about 70 seconds in the novel arm.

These results show that the Control Group animals performed worse than the Reference Group and the SIA-supplemented Group animals. Thus, these Y-maze results indicate that free sialic acid may play a critical play role in acquisition and storage of spatial memories.

### Long-term Potentiation Testing

Long-term potentiation (LTP) measurements were performed in each of the three groups, i.e., the Control Group, the SIA-supplemented Group and the Reference Group. The testing was done while the animals were alive and at postnatal day 110 (PND110). Postnatal day 110 in rats equates to approximately 10 to 20 years of age in the lifespan of a human. Since hippocampus is a key brain structure related to LTP memory, experiments were done in order to determine the functionality of hippocampus *in vivo.* Long-term potentiation (LTP) is considered as the electrophysiological base of memory. In these experiments, a higher response indicates better memory potential.

The test was performed after implantation of electrodes in the hippocampal CA3-CAl areas of the rats in each group. There were seven rats per group. Each animal had 4 electrodes inserted and LTP measurements were taken from each electrode. Synaptic field potentials in the CAl area were evoked by paired (40 ms of interval) 100 µs, square, biphasic (negative-positive) pulse applied to Schaffer collaterals. For each animal, the stimulus intensity was set well below the threshold for evoking a population spike, usually 30-40% of the intensity necessary for evoking a maximum field excitatory postsynaptic potential ("fEPSP') response. An excitatory postsynaptic potential (EPSP) is a temporary depolarization of postsynaptic membrane potential caused by the flow of positively charged ions into the postsynaptic cell as a result of opening of ligand-gated ion channels.

To evoke LTP, the following high frequency stimulation (HFS) protocol was used: each animal was subjected to five 200 Hz, 100 ms trains of pulses at a rate of l/s. These trains were presented 6 times in total, at intervals of 1 minutes. After the HFS protocol, fEPSPs were recorded again for 30 minutes. Additional recordings were carried out for 15 minutes during the 3 following days.

### Results of Long-term Potentiation Testing

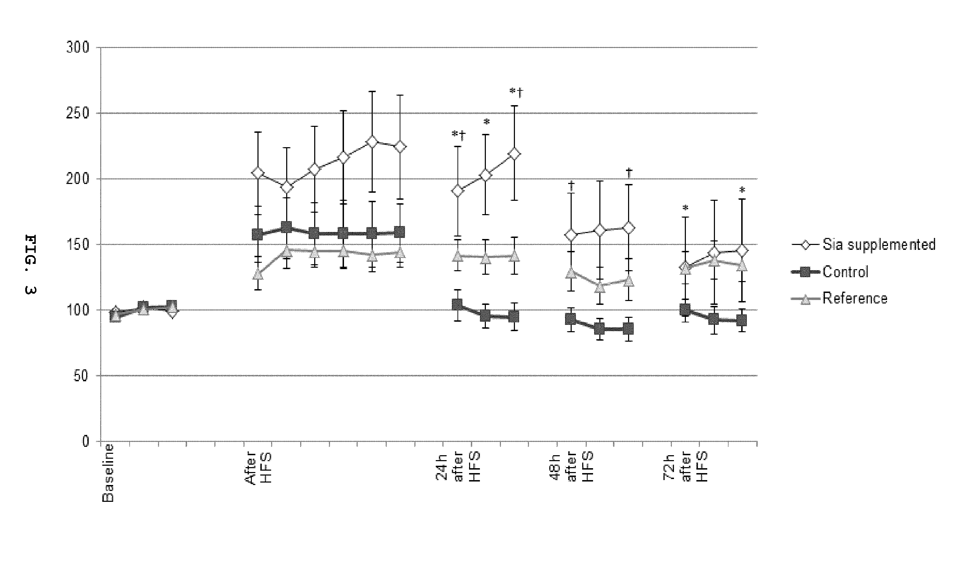

FIG. 3 depicts the results of the Long-term Potentiation Testing. FIG. 3 illustrates the results of induction of LTP by the use of a high-frequency stimulation (HFS) protocol. Results correspond to the slope of the field excitatory postsynaptic potential ("fEPSP') evoked by a second electronic pulse (which took place 40 ms after a first pulse). Significant differences (p < 0.05) are indicated by the following signs: "*" indicates the differences between the SIA-supplemented Group and the Control Group; and "†" indicates the differences between the Reference Group and the Control Group. Average baseline fEPSP for each group was assigned the value of 100, and the rest of the fEPSP were referred to that value.

Rats in the Reference Group had an average fEPSP of about 145 after high-frequency stimulation fEPSP slope. Twenty-four hours, forty-eight hours, and seventy-two hours after high-frequency stimulation, fEPSP slope remained elevated and did not return to pre-stimulation levels, averaging about 140, 125, and 135, respectively.

After high-frequency stimulation fEPSP slope averaged about 220 for rats in the SIA-supplemented Group. Twenty-four hours, forty-eight hours, and seventy-two hours after high-frequency stimulation, fEPSP slope remained elevated and did not return to pre-stimulation levels, averaging about 205, 160, and 140, respectively.

After high-frequency stimulation fEPSP slope averaged about 160 for Control rats. Twenty-four hours, forty-eight hours, and seventy-two hours after high-frequency stimulation, fEPSP slope returned to basal levels, averaging about 100, 90, and 95, respectively.

These results show that the stimulus evoked the strongest response in the SIA-supplemented Group. The response in the SIA-supplemented Group was better than in the Reference Group, and this response remained at high levels even 72 hours post stimulus. To the contrary, the hippocampal stimulation response of the Control Group was small and returned to basal levels just 24 hours after stimulation. This data supports the results from the Y-maze test, linking the observed behavior to its physiological basis.

### Conclusions from the Experiments

In the spatial task, i.e., the Y-maze, free sialic acid-supplemented animals performed better and similarly to reference animals, when compared to non-supplemented control animals. Moreover, when LTP response in hippocampus was evaluated *in vivo,* free sialic acid-supplemented animals reached significantly higher LTP response levels than those of the control group (which received a low free sialic acid input during lactation.) This experimental data confirms the crucial role of free sialic acid in the developing brain and shows that the beneficial effects of free sialic acid on learning and memory abilities are sustained in that they remain in adulthood or adolescence.

### EXAMPLE 2

In order to establish the role of sialylated oligosaccharides in cognition, rat pups were supplemented with 6'-sialyllactose (6'-SL) from postnatal day 3 (PND3) and their learning and memory abilities were analyzed later in life. Rat performance in a classical spatial cognitive task (Y-maze), Novel object Recognition, protocol of spatial learning in the novel Intellicage^{®} system, and Morris water maze were carried out once the animals reached 12 and 14 months of age. LTP response in hippocampus was also evaluated *in vivo* once the animals reached 11 months of age. The Y-maze, Object Recognition and the IntelliCage^{®} allow the determination of spatial orientation and memory capabilities of experimental animals. The same female rats performed all the tests.

In order to bypass milk containing the highest levels of sialylated structures, two cohorts of pregnant Sprague-Dawley rats were utilized: the foster mothers in the first cohort and the biological mothers in the second cohort. The first cohort contained 12 mothers. The first cohort gave birth 16 days before the second cohort.

The second cohort contained 12 mothers. Once the pups born to the second cohort were 3 days old, they were removed from their biological mothers and given to foster mothers in the first cohort after removing their own pups. Each foster mother received 5 male and 5 female pups and fed them until weaning at day 21. Similarly to example 1, the milk of these foster mothers is low in sialic acid. Some of the pups died before weaning, but no relationship between survival and diet was observed.

The pups from the second cohort were divided into three groups and given the following designations: the 6'-SL supplemented group, the NeuAc supplemented group and the control group. Initially, each group comprised 20 male pups. Due to premature deaths there were a total of 14 pups in the 6'-SL supplemented group at the end of the lactation. The 6'-SL supplemented group received 6'-SL supplementation (8 doses/day) in order to reach the same sialic acid levels as normally reared pups. The amount of 6'-SL given to the 6'-SL supplemented group was selected to follow the change in concentration of sialic acid in rat milk, ranging from 1.5 g/Kg body weight on day 3 to 0.5 g/Kg body weight at weaning. The maximum amount of sialic acid given to a pup was a maximum of 2 mg/Kg on day 8.

There were a total of 16 male pups in the NeuAc supplemented group at the end of lactation. The NeuAc supplemented group received NeuAc supplementation in order to reach the same sialic acid levels as normally reared pups. The amount of NeuAc given to the NeuAc supplemented group was selected to follow the change in concentration of sialic acid in rat milk, ranging from 1.5 g/Kg body weight on day 3 to 0.5 g/Kg body weight at weaning. The maximum amount of sialic acid given to any pup was 2 mg/Kg on day 8,

The Control group of rat pups were fed milk containing low free sialic acid content and received only vehicle as a supplement. The composition of the vehicle was 0.9% NaCl in water. There were 14 pups in the Control group.

All pups in each of the three groups utilized in these examples were male. The pups in each group weaned after 21 days. Once weaned, all the animals were fed standard chow diet regardless of the experimental group to which they belonged.

### Long-term Potentiation Testing

Long-term potentiation (LTP) measurements were performed in each of the three groups, i.e., the 6'-SL Supplemented Group, the NeuAc -Supplemented Group and the Control Group. The testing was done while the animals were alive and 11 months old, which is about age 40 in human years. Experiments were done in order to determine the functionality of hippocampus *in vivo.* In these experiments, a higher response indicates better memory potential.

The test was performed after implantation of electrodes in the hippocampal CA3-CAl areas of the rats in each group. The tests were performed once on all the rats in each of the three groups. Synaptic field potentials in the CAl area were evoked by paired (40 ms of interval) 100 µs, square, biphasic (negative-positive) pulse applied to Schaffer collaterals. For each animal, the stimulus intensity was set well below the threshold for evoking a population spike, usually 30-40% of the intensity necessary for evoking a maximum field excitatory postsynaptic potential ("fEPSP') response. An excitatory postsynaptic potential (EPSP) is a temporary depolarization of postsynaptic membrane potential caused by the flow of positively charged ions into the postsynaptic cell as a result of opening of ligand-gated ion channels.

To evoke LTP, the following high frequency stimulation (HFS) protocol was used: each animal was subjected to five 200 Hz, 100 ms trains of pulses at a rate of l/s. These trains were presented 6 times in total, at intervals of 1 minutes. After the HFS protocol, fEPSPs were recorded again for 30 minutes. Additional recordings were carried out for 15 minutes during the 3 following days.

### Results of Long-term Potentiation Testing

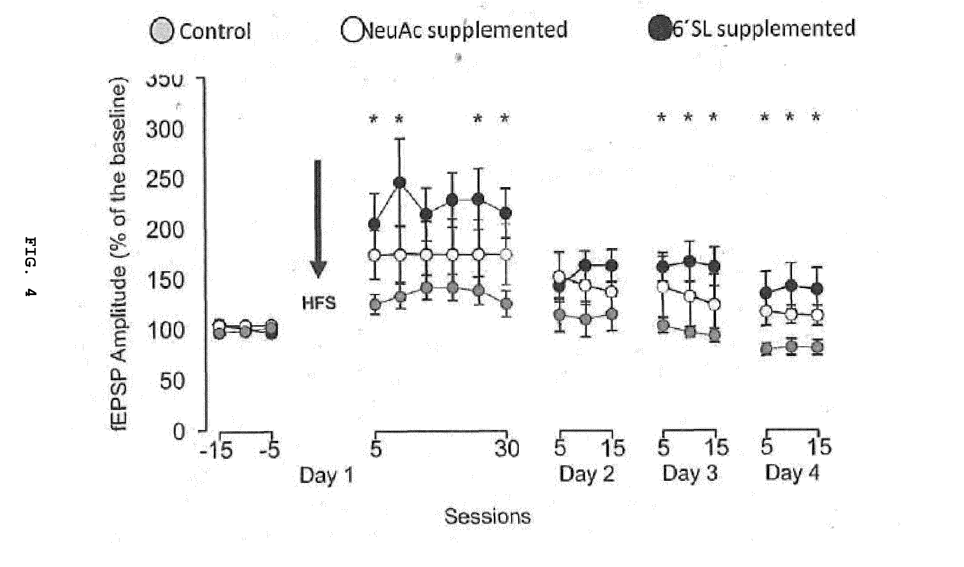

FIG. 4 depicts the results of the Long-term Potentiation Testing. FIG. 4 illustrates the results of induction of LTP by the use of a high-frequency stimulation (HFS) protocol. LTP evolution was followed for up to 4 days. The three groups of animals presented a significant induction on the LTP on the first day, and such induction was lost in Control rats after the second day, while remaining in the 6'SL and NeuAc supplemented rats.

In FIG. 4, the "*" symbol indicates a significant difference between the fEPSP value for the 6'SL supplemented rats and the fEPSP value for Control group rats. Thus, for the most part fEPSP values evoked after the HFS train were significantly larger for 6'-SL supplemented animals in comparison with values collected from the Control group. The data in FIG. 4 are represented as Mean ± SEM collected from ten animals per group.

FIG 4. shows the data collected by experimentally evoked LTP in the three groups of animals before and after the high-frequency stimulation (HFS) train. The HFS train is indicated by the downward arrow. The HFS was presented after 15 min of baseline recordings. The baseline was recorded at 15, 10, and 5 minutes before HFS.

These results show that the stimulus evoked the strongest response in the 6'SL Supplemented Group. The response in the 6'SL -Supplemented Group was better than in the Control Group and the NeuAc -Supplemented Group. Additionally, this response remained at elevated levels even 4 days post stimulus. To the contrary, the hippocampal stimulation response of the Control Group was small and returned to basal levels within days after stimulation.

### Novel Object Recognition Testing

Perirhinal cortex and hippocampus are involved in the novel object recognition task. Novel Object Recognition Testing was performed when the rats were 13 months old, which corresponds to 45-50 years old for humans. Eight animals were tested.

During a habituation phase of the test, the rats were allowed to explore an "arena" for 20 minutes per day for 3 consecutive days. The arena was a black, open plastic chamber. Next, an acquisition phase took place wherein the animals interfaced with two different objects. These object are defined as "familiar." The rats were allowed to the arena while the familiar objects were present for 10 minutes. One day after the acquisition phase, a retention test was performed on the rats, wherein one of the familiar objects was replaced by a new object. The new object is defined as "novel." The rats were allowed to explore the arena for 5 minutes during the retention test. In theory, animals that remember familiar objects will spend more time exploring the novel object than the familiar one.

### Results of Novel Object Recognition Testing

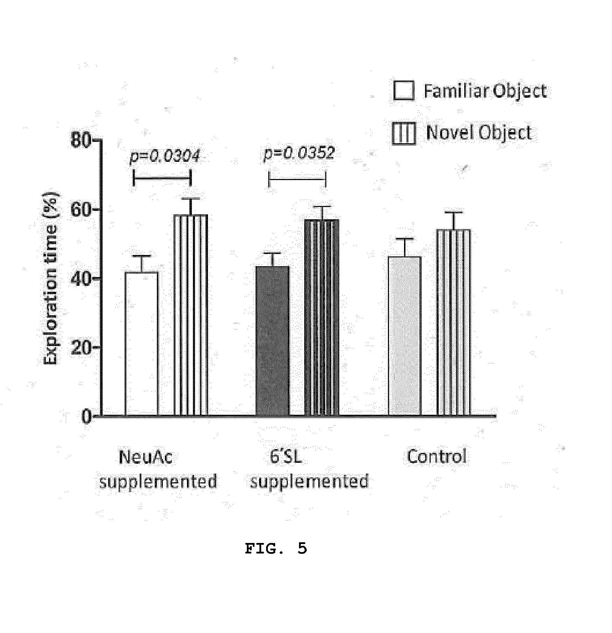

FIG. 5 depicts the results of Novel Object Recognition tests. The solid bar depicts the percentage of time the animals spent exploring the familiar object. The striped bar depicts the percentage of time the animals spent exploring the novel object. One way ANOVA and Student's t-test were used to determine these values. In FIG. 5, p-values are provided. P-values less than 0.5 denote statistically significant differences in time spent in exploring a novel object verses a familiar object. Rats in the NeuAc-Supplemented and 6'SL-Supplemented groups spent significantly more time visiting the novel object than the familiar one.

### Y-maze Testing

A Y-maze test was performed when the rats were 14 months old, which corresponds to 50-55 years old in humans. Eight rats in each group were tested. The Y-maze testing was performed in two phases: an Acquisition Trial and a Retention Trial. Rats in each group were evaluated in the Y-maze.

During the Acquisition Trial, one of the three arms of the Y-maze was blocked, and animals were allowed to explore the other two arms for 15 minutes. The Retention Trial took place 4 hours after the Acquisition Trial. During the Retention Trial, all three arms were open and animals were allowed to explore for 5 minutes. The amount of time each animal spent at the start of the Y-maze and each arm was evaluated. By spending a longer time visiting the previously blocked arm during the Retention Trial, an animal displays a normal interest in novelty and an apparent ability to remember which arms it has previously visited.

### Results of Y-maze Testing

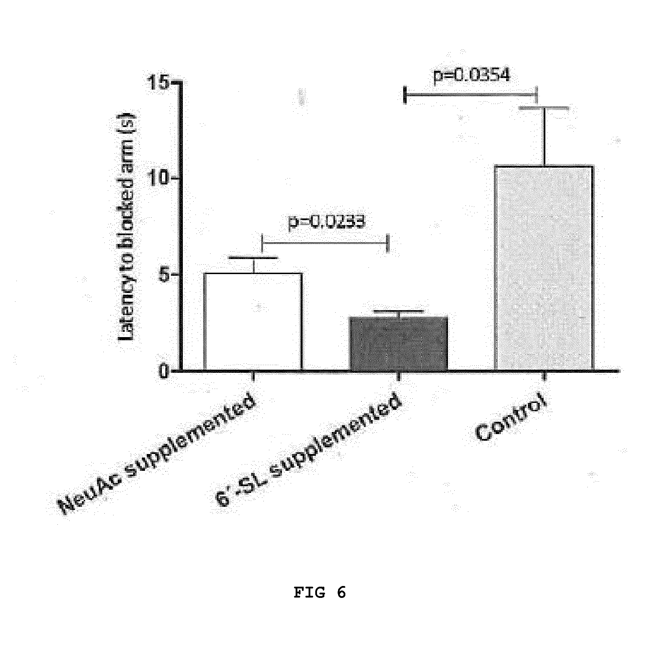

FIG. 6 depicts the latency values to blocked arm. During the Retention Trial, rats that visited the novel arm first are said to have a low latency to the blocked arm. As FIG. 6 shows, 6'-SL supplemented and NeuAc supplemented groups showed lower values of latency to the blocked arm in comparison with Control group. Differences between 6'-SL and control groups were statistically significant.

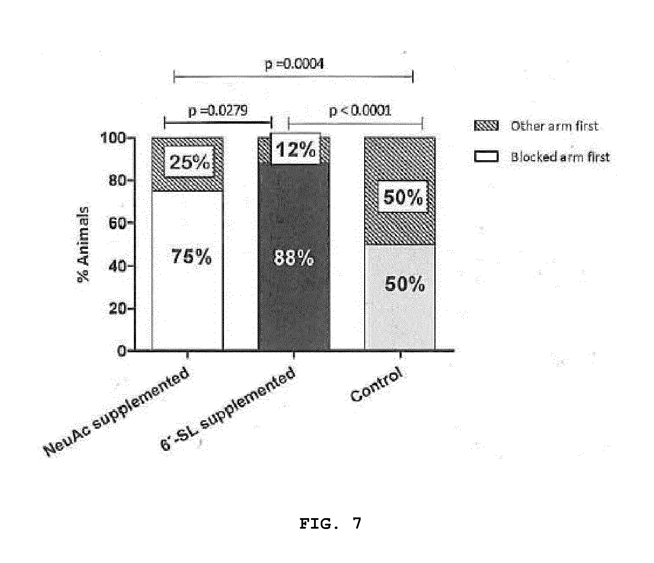

FIG. 7 depicts the percentage of animals in each group that chose either the previously blocked arm or familiar arms first. The striped bar indicates the percentage that chose the familiar arm first and the solid bar indicates the number of animals that chose the previously blocked arm first. The percentage of NeuAc-supplemented and 6'SL supplemented groups visiting the previously blocked arm first was significantly higher than that of the control animals.

### Intellicage^{®} Testing

IntelliCage^{®} (NewBehavior AG) is a computer-based, fully automated testing apparatus used to analyze spontaneous and learned behavior. Eight rats in each group were tested. The rats were 14-15 months old, which corresponds to 55-60 years old for humans, when these tests were performed. Animals remain in the cage during several days and behavior is recorded during the entire testing period. Different paradigms can be tested by restricting water access to specific places and time periods. Each animal is assigned a corner where it can access water. Access to the water is only allowed during discrete time periods. Thus, each animal must learn when access to water bottles is allowed and which corner, out of four, has been assigned to it. Access to the corner is controlled by a door that opens only when the assigned rat enters its nose in a hole. Doors do not open if the rat visits a corner that has not been assigned to it. Access to non-assigned corners remain blocked except during two 90 min periods during the night.

### Results of Intellicage^{®} Testing

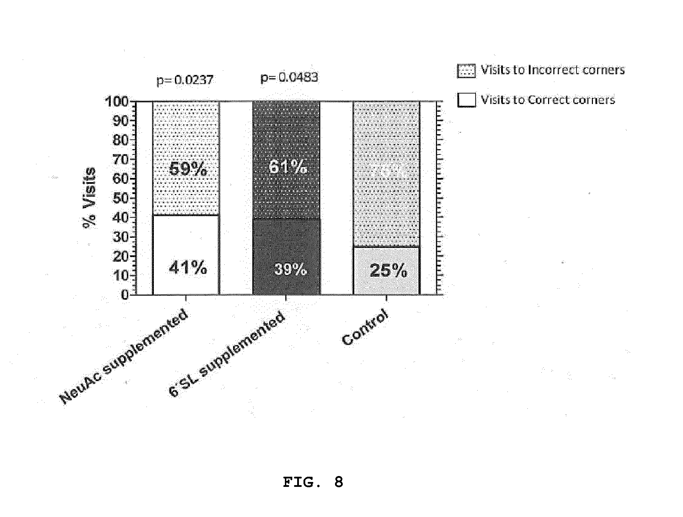

FIG. 8 depicts the percentage of visits to the correct corner and the percentage of visits to the incorrect corner for rats in each group. Visiting the correct corner allows the animal to receive water. As shown in FIG. 8, results show that rats in the NeuAc-supplemented and 6'-SL supplemented groups performed better compared to the Control group. Rats in the NeuAc-supplemented and 6'-SL supplemented groups seemed to learn faster than the Control group.

### Conclusions from the Experiments

In the spatial tasks, i.e., the Y-maze, Intellicage^{®} testing, and Novel Object Recognition testing sialic acid-supplemented animals performed better and similarly to reference animals, when compared to non-supplemented control animals. Moreover, when LTP response in hippocampus was evaluated *in vivo*, 6'SL-supplemented animals reached significantly higher LTP response levels than those of the control group (which received a low sialic acid input during lactation.) Supporting the results of the LTP, data obtained in the novel object recognition show that animals in the NeuAc supplemented and 6'SL supplemented groups distinguish better between the familiar and novel object implying better learning skills than in the Control group. Overall, the experimental data confirms the crucial role of sialic acid in the developing brain and shows that the beneficial effects of sialic acid on learning and memory abilities remain in adulthood.

The present disclosure should also be understood as including the following as embodiments. 1. A method of achieving sustained memory improvement in an infant, comprising: administering an effective amount of sialic acid to the infant until at least 90 days after birth, wherein the amount of sialic acid administered decreases as the infant ages; and whereby the administration results in enhanced brain function in the infant. 2. The method of 1, whereby the administration of sialic acid results in sustained memory improvement. 3. The method of 1 or 2, whereby the amount of sialic acid administered decreases on a week-to-week basis as the infant ages. 4. The method of 1 or 2, whereby the amount of sialic acid administered decreases on a month-to-month basis as the infant ages. 5. A method of achieving sustained memory improvement comprising: administering an effective amount of sialic acid to a preterm infant for at least 3 months after the birth of the infant. 6. The method of 5, whereby the memory of the infant is improved. 7. A method of achieving a sustained improvement in learning abilities in an infant comprising: administering an effective amount of sialic acid to the infant for at least 3 months after the birth of the infant. 8. The method of 7, whereby the learning abilities of the infant are improved. 9. The method of any one of 2, 6, or 8, wherein the improvement is sustained at least until the infant reaches childhood. 10. The method of any one of 1-9, wherein the amount of sialic acid administered on an as-fed basis is from 60 mg/kg bodyweight (of the infant)/day to 250 mg/kg bodyweight (of the infant)/day. 11. The method of any one of 1-9, wherein the amount of sialic acid administered on an as-fed basis is from 60 mg/kg bodyweight (of the infant)/day to 400 mg/kg bodyweight (of the infant)/day. 12. The method of any one of 1-9, wherein the amount of sialic acid administered on an as-fed basis is at least 60 mg/kg bodyweight (of the infant)/day. 13. The method of any one of 1-9, wherein the amount of sialic acid administered on an as-fed basis is: at least 250 mg/kg bodyweight (of the infant)/day, through day 5 postpartum; at least 200 mg/kg bodyweight (of the infant)/day after day 5 postpartum and through day 15 postpartum; and at least 60 mg/kg bodyweight (of the infant)/day after day 15 postpartum. 14. The method of any one of 1-9, wherein the amount of sialic acid administered on an as-fed basis is: from 200 mg/kg bodyweight (of the infant)/day to 400 mg/kg bodyweight (of the infant)/day through day 5 postpartum; from 60 mg/kg bodyweight (of the infant) /day to 250 mg/kg bodyweight (of the infant) /day after day 5 postpartum and through day 15 postpartum; and from 60 mg/kg bodyweight (of the infant)/day to 100 mg/kg bodyweight (of the infant)/day after day 15 postpartum. 15. The method of any one of 1-14, wherein the composition is administered with a food product selected from the group consisting of mammalian food products, synthetic infant formula, semi-synthetic infant formula, human milk fortifier, and human breast milk. 16. A method of achieving sustained memory improvement in an infant, comprising: combining a composition having sialic acid with an infant formula that is low in sialoglycoconjugated compounds; and administering the combination to the infant, whereby the administration of the combination results in sustained memory improvement. 17. The method of 16, wherein the improvement is sustained at least until the infant reaches childhood. 18. The method of 16 or 17, wherein the concentration of sialic acid in the combination on an as-fed basis is: from 1000 mg/L to 2000 mg/L through day 5 postpartum; from 500 mg/L to 1500 mg/L after day 5 postpartum through day 15 postpartum; and from 0 mg/L to 1000 mg/L after day 15 postpartum. Item 19. The method of 16 or 17, wherein the concentration of sialic acid in the combination on an as-fed basis is: 1500 mg/L through the first 5 days postpartum; 1000 mg/L after day 5 postpartum and through day 15 postpartum; and 500 mg/L after day 15 postpartum. 20. The method of any one of 1-19, wherein the infant formula is soy-based. 21. The method of any one of 1-20, wherein the sialic acid is selected from the group consisting of 6'-sialyllactose, 3'-sialyllactose, 6'-sialyllactosamine, 3'-sialyllactosamine, and combinations thereof. 22. The method of any one of 1-20, wherein the sialic acid is bound to an acidic milk oligosaccharide acid. 23. The method of any one of 1-20, wherein the sialic acid is 6'-sialyllactose. 24. The method of 21, wherein the sialic acid is free sialic acid. 25. The method of any one of 1-24, whereby the administration of sialic acid results in sustained memory improvement at least until the infant reaches adolescence. 26. The method of any one of 1-25, whereby the administration of sialic acid results in sustained memory improvement at least until the infant reaches adulthood. 27. The method of any one of 1-26, wherein the infant is not malnourished at the time the sialic acid is administered. 28. The method of any one of 15 or 16-27, wherein the food product or the combination is administered orally. 29. The method of any one of 15 or 16-27, wherein the food product or the combination is delivered enterally. 30. The method of any one of 1-29, further comprising administering an effective amount of sialic acid to the infant until at least 6 months after birth.

To the extent that the term "includes" or "including" is used in the specification or the claims, it is intended to be inclusive in a manner similar to the term "comprising" as that term is interpreted when employed as a transitional word in a claim. Furthermore, to the extent that the term "or" is employed (e.g., A or B) it is intended to mean "A or B or both." When the applicants intend to indicate "only A or B but not both" then the term "only A or B but not both" will be employed. Thus, use of the term "or" herein is the inclusive, and not the exclusive use. See Bryan A. Garner, A Dictionary of Modern Legal Usage 624 (2d. Ed. 1995). Also, to the extent that the terms "in" or "into" are used in the specification or the claims, it is intended to additionally mean "on" or "onto."

While the present application has been illustrated by the description of embodiments thereof, and while the embodiments have been described in considerable detail, it is not the intention of the applicants to restrict or in any way limit the scope of the appended claims to such detail. Additional advantages and modifications will readily appear to those skilled in the art. Therefore, the application, in its broader aspects, is not limited to the specific details, the representative compositions and processes, and illustrative examples shown and described. Accordingly, departures may be made from such details without departing from the spirit or scope of the applicant's general inventive concept.

## Claims

1. Sialic acid for use in enhancing brain function in an infant wherein sialic acid is administered until at least 90 days after birth, and wherein the amount of sialic acid administered decreases as the infant ages.

2. The compound of claim 1, wherein the enhanced brain function is sustained memory improvement.

3. The compound of claim 1 or claim 2, whereby the amount of sialic acid administered decreases on a week-to-week basis as the infant ages.

4. The compound of claim 1 or claim 2, whereby the amount of sialic acid administered decreases on a month-to-month basis as the infant ages.

5. Sialic acid for use in achieving sustained memory improvement in a preterm infant, wherein the sialic acid is administered to the preterm infant for at least 3 months after the birth of the infant.

6. Sialic acid for use in achieving sustained improvement in learning abilities in an infant, wherein the sialic acid is administered to the infant for at least 3 months after the birth of the infant.

7. The compound of any one of the preceding claims, wherein the improvement is sustained at least until the infant reaches childhood.

8. The compound according to of any one of claims 1-7, wherein the amount of sialic acid administered on an as-fed basis is at least 60 mg/kg bodyweight (of the infant)/day.

9. The compound according to claim 8, wherein the amount of sialic acid administered on an as-fed basis is from 60 mg/kg bodyweight (of the infant)/day to 400 mg/kg bodyweight (of the infant) /day.

10. The compound according to claim 9, wherein the amount of sialic acid administered on an as-fed basis is from 60 mg/kg bodyweight (of the infant)/day to 250 mg/kg bodyweight (of the infant) /day.

11. The compound according to any one of claims 1-7, wherein the amount of sialic acid administered on an as-fed basis is:
at least 250 mg/kg bodyweight (of the infant)/day, through day 5 postpartum;
at least 200 mg/kg bodyweight (of the infant)/day after day 5 postpartum and through day 15 postpartum; and
at least 60 mg/kg bodyweight (of the infant)/day after day 15 postpartum.

12. The compound according to of any one of claims 1-7, wherein the amount of sialic acid administered on an as-fed basis is:
from 200 mg/kg bodyweight (of the infant)/day to 400 mg/kg bodyweight (of the infant)/day through day 5 postpartum;
from 60 mg/kg bodyweight (of the infant)/day to 250 mg/kg bodyweight (of the infant)/day after day 5 postpartum and through day 15 postpartum; and
from 60 mg/kg bodyweight (of the infant)/day to 100 mg/kg bodyweight (of the infant)/day after day 15 postpartum.

13. The compound according to of any one of claims 1-12, wherein the compound is administered with a food product selected from the group consisting of mammalian food products, synthetic infant formula, semi-synthetic infant formula, human milk fortifier, and human breast milk.

14. A composition for use in achieving sustained memory improvement in an infant, wherein the composition comprises a combination of sialic acid and an infant formula that is low in sialoglycoconjugated compounds.

15. The composition of claim 14, wherein the improvement is sustained at least until the infant reaches childhood.

16. The composition of claim 14 or claim 15, wherein the concentration of sialic acid in the combination on an as-fed basis is:
from 1000 mg/L to 2000 mg/L through day 5 postpartum;
from 500 mg/L to 1500 mg/L after day 5 postpartum through day 15 postpartum; and
from 0 mg/L to 1000 mg/L after day 15 postpartum.

17. The composition of claim 14 or claim 15, wherein the concentration of sialic acid in the combination on an as-fed basis is:
1500 mg/L through the first 5 days postpartum;
1000 mg/L after day 5 postpartum and through day 15 postpartum; and
500 mg/L after day 15 postpartum.

18. The composition of any one of claims 14-17, wherein the infant formula is soy-based.

19. The compound of any one of claims 1-13 or the composition of any one of claims 14 to 18, wherein the sialic acid is selected from the group consisting of 6'-sialyllactose, 3'-sialyllactose, 6'-sialyllactosamine, 3'-sialyllactosamine, and combinations thereof.

20. The compound of any one of claims 1-13 or the composition of any one of claims 14 to 19, wherein the sialic acid is bound to an acidic milk oligosaccharide acid.

21. The compound of any one of claims 1-13 or the composition of any one of claims 14 to 20, wherein the sialic acid is 6'-sialyllactose.

22. The compound or composition of claim 21, wherein the sialic acid is free sialic acid.

23. The compound or composition of any one of claims 1-22, whereby the administration of sialic acid results in sustained memory improvement at least until the infant reaches adolescence.

24. The compound or composition of any one of claims 1-23, whereby the administration of sialic acid results in sustained memory improvement at least until the infant reaches adulthood.

25. The compound or composition of any one of claims 1-24, wherein the infant is not malnourished at the time the sialic acid is administered.

26. The compound or composition of any one of claims 1-25, wherein the compound or the composition is administered orally.

27. The compound or composition of any one of claims 1-25, wherein the or the composition is delivered enterally.

28. The compound or composition of any one of claims 1-27, further comprising administering an effective amount of sialic acid to the infant until at least 6 months after birth.
